# EUROPEAN PATENT APPLICATION

(11) **EP 4 772 612 A1**
(43) Date of publication of application: **08.07.2026**
(21) Application number: 24921785.2
(22) Date of filing: 20.12.2024
(51) Int. Cl.: C12N 5/0775, C12N 5/077, A61L 27/38, A61L 27/20, A61L 27/24, A61L 27/52

(54) **USE OF STEM CELLS OR TRANSGENIC STEM CELLS**

(30) Priority: 01.02.2024 CN 202410137199
(71) Applicant: Harbin Longhui Stem Cell Biotechnology Co., Ltd, Harbin, Heilongjiang 150086 (CN)
(72) Inventor: QU, Fujun, Harbin, Heilongjiang 150025 (CN); WANG, Ming, Harbin, Heilongjiang 150025 (CN); XU, Guangyi, Harbin, Heilongjiang 150087 (CN); XU, Jing, Harbin, Heilongjiang 150028 (CN)
(74) Representative: Patent 42
(86) International application number: PCT/CN2024/141195
(87) International publication number: WO 2025/161763

(57) **Abstract**

The present invention relates to an application of stem cells or transgenic stem cells, and specifically, the stem cells or transgenic stem cells are used for preparing a stem cell gel solution or tissue-engineered cartilage. Finally obtained grafts are capable of expressing active proteins. These proteins induce differentiation of a host's own stem cells into chondrocytes. This process accelerates graft integration with host tissue.

## Description

### TECHNICAL FIELD

The present invention relates to the technical field of biomedicine, and in particular to an application of stem cells or transgenic stem cells.

### BACKGROUND

Cartilage injury is a common clinical orthopedic disease usually caused by joint sprains, a local external impact, or any other factor, and pathological characteristics include articular cartilage degeneration and destruction, subchondral bone sclerosis, reactive hyperplasia at joint margins and subchondral bone, and osteophyte formation. Currently, there are seven main clinical therapies for cartilage injury, and specific therapies and their defects are as follows:
1. Palliative therapy: Also known as joint lavage, the palliative therapy is a common clinical therapy of mainly removing the damaged cartilage without replacing it. Defects: As a conservative therapy, the palliative therapy requires only removing damaged cartilage fragments and removing osteophytes, improves mobility, relieves symptoms, and eases pain, but has no reparative effect on cartilage injury, with very poor curative effect.
2. Reparative therapy: Also known as the microfracture technique, the reparative therapy is a common clinical therapy that requires debriding the cartilage in a defect area and drilling holes in the subchondral bone to form a blood clot and induce spontaneous repair of stem cells derived from bone marrow. Defects: an injured site is filled with only fibrous tissue without repairing cartilage, and the curative effect is poor.
3. Artificial joint replacement surgery: This is a common clinical therapy involving surgical replacement with artificial metal or non-metal joints. Defects: Significantly different from human joints, an implanted artificial joint is poorly integrated with a joint of the human body, such that a patient suffers from persistent pain of varying degrees, and faces high risks of prosthesis infection, loosening, fracture, and dislocation.
4. Autologous chondrocyte implantation (ACI): Having an unsatisfactory clinical effect, ACI requires transplanting autologous cartilage tissue to an injured site. Defects: As a therapy of treating an injury by creating another, ACI requires two joint surgeries (at least two surgeries on two joint sites); chondrocytes are limited and prone to aging and phenotypic changes; and ACI is an individualized therapy and cannot be commercially promoted on a large scale.
5. Transplantation of tissue-engineered cartilage with autologous chondrocytes cultured in vitro: The clinical effect is also unsatisfactory, and the case is similar for a MACI product approved for marketing in the United States. Defects are the same as those of the 4th therapy.
6. Osteochondral allograft (OCA) transplantation: Rarely used in clinical practice, OCA requires transplanting cartilage from another individual. Defects: Due to difficulties in obtaining donor sources, OCA is almost impossible to implement by treating one's own injury by causing injury to another.
7. Implantation of allogeneic umbilical cord blood-derived mesenchymal stem cells: This therapy is rarely used in clinical practice due to high clinical risks, and for example, the case is similar for a Cartistem product approved for marketing in South Korea. Defects: Umbilical cord blood-derived mesenchymal stem cells are implanted at a cartilage injury site, and are transformed into chondrocytes at the cartilage injury site in a limited manner; and although having good cartilage regeneration effect, this therapy cannot achieve complete repair due to unknown allogeneic reactions.

Among the above seven therapies, the 1st to 3rd therapies are traditional therapies are widely used at home and abroad, and have no reparative effect on cartilage injury, and demonstrate unsatisfactory therapeutic effects, belonging to conservative and palliative therapies with many drawbacks. The 4th to 7th therapies as modern biological therapies are significantly improved in curative effects compared with traditional therapies, and are capable of partially repairing damaged cartilage, but have drawbacks such as significant adverse reactions, low integration, inability to completely repair cartilage injury, and failure to achieve complete cure.

Furthermore, Qu Fujun et al. constructed BMP7 gene-transfected tissue-engineered cartilage using BMP7 gene-transfected chondrocytes with collagen-fibrin gel as a three-dimensional scaffold material (Qu Fujun, Hou Yi, Liu Liling, et al. Application of BMP7 gene-transfected chondrocytes to construct tissue-engineered cartilage [J]. Chinese Journal of Tissue Engineering Research, 2006, 10 (13): 59-61), and transplanted BMP7 gene-transfected tissue-engineered cartilage to repair rabbit knee joint cartilage defects (Qu Fujun, Sun Hua, Feng Nianping, et al. Observation on repairing rabbit knee joint cartilage defects with BMP7 gene-transfected tissue-engineered cartilage [J]. Chinese Journal of Tissue Engineering Research, 2008, 27 (1): 48-52). This therapy for repairing cartilage injury by transplantation suffers from limitations in a seed cell source of tissue-engineered cartilage tissue, and is not suitable for repairing large-area cartilage injury. No matter whether seed cells are derived from autologous or allogeneic sources, this therapy resorts to treating an injury by creating another and is not suitable for large-scale industrial production. Therefore, it is difficult to develop seed cell-derived drugs for clinical use.

### SUMMARY

In view of the above, an objective of the present invention is to provide an application of stem cells or transgenic stem cells in treatment of cartilage injury, so as to solve a series of problems in the prior art such as insufficient seed cell sources of tissue-engineered cartilage, inability of in vitro massive expansion, susceptibility to aging after expansion, and poor integration of a tissue-engineered cartilage graft with host tissue. A technical solution of the present invention is as follows:
The present invention relates to an application of stem cells or transgenic stem cells in preparing a stem cell gel solution or tissue-engineered cartilage.

Preferably, the stem cells are derived from umbilical cord blood, umbilical cords, placentas, bone marrow, or fat, and further preferably, the stem cells are umbilical cord blood-derived mesenchymal stem cells or bone-derived marrow mesenchymal stem cells.

Preferably, the gene is selected from at least one of a bone morphogenetic protein (BMP) gene, a transforming growth factor-β (TGF-β) gene, an insulin-like growth factor (IGF) gene, a fibroblast growth factor (FGF) gene, and a SOX9 gene; and the BMP gene is preferably BMP7 and/or BMP2.

Preferably, a method for preparing a stem cell gel solution or constructing tissue-engineered cartilage using stem cells includes the following steps:
step 1: isolating and culturing stem cells;
step 2: inducing differentiation of the stem cells using an active protein; and
step 3: preparing a stem cell gel solution using the differentiated stem cells, or constructing a type III collagen 3D scaffold in vitro using the stem cells obtained in the step 2 to finally form tissue-engineered cartilage.

Preferably, a method for preparing a stem cell gel solution or constructing tissue-engineered cartilage using transgenic stem cells includes the following steps:
step 1: constructing a recombinant plasmid containing a gene of interest;
step 2: isolating and culturing stem cells;
step 3: transfecting the stem cells cultured in the step 2 with the recombinant plasmid constructed in the step 1 to obtain stem cells transfected with the gene of interest;
step 4: inducing differentiation of the stem cells transfected with the gene of interest using an active protein; and
step 5: preparing a stem cell gel solution using the differentiated stem cells, or constructing a type III collagen 3D scaffold in vitro using the stem cells obtained in the step 4 to finally form tissue-engineered cartilage.

Preferably, the constructing a recombinant plasmid containing a gene of interest in the step 1 includes:
step 1-1: obtaining a pBlue-gene of interest plasmid;
step 1-2: treating the plasmid through double digestion, and then extracting and purifying a gene fragment;
step 1-3: similarly treating a pcDNA3.1 vector through double digestion, and then extracting and purifying a vector fragment;
step 1-4: ligating the purified gene fragment and the vector fragment to form a pcDNA3.1-gene of interest vector plasmid; and
step 1-5: introducing the plasmid into DH5α competent cells for amplification, and finally extracting and purifying an amplified product to obtain the required plasmid.

Preferably, the gene of interest is selected from at least one of a bone morphogenetic protein (BMP) gene, a transforming growth factor β (TGF-β) gene, an insulin-like growth factor (IGF) gene, a fibroblast growth factor (FGF) gene, and a SOX9 gene; and the BMP gene is preferably BMP7 and/or BMP2.

Preferably, the stem cells adopted in the step 1 or the step 2 are derived from umbilical cord blood, umbilical cords, placentas, bone marrow, or fat, and further preferably, the stem cells are umbilical cord blood-derived mesenchymal stem cells or bone-derived marrow mesenchymal stem cells.

Preferably, the isolating and culturing stem cells includes:
step 2-1: adding obtained stem cell-derived tissue into a DMEM culture medium, mixing uniformly, and centrifuging;
step 2-2: removing a fat layer from a centrifugate, mixing the centrifugate uniformly with a percoll separation solution with a density of 1.073 g/ml at a volume ratio of 1:2, and centrifuging;
step 2-3: isolating monocytes, washing sequentially, adding a stem cell culture medium to suspend the cells, inoculating the cells into a culture flask at a cell density of 5×10⁶ cells/ml with an inoculation volume of 3 ml, culturing the cells at 37°C with 5% CO₂ for 3-4 days, discarding non-adherent cells, and replacing with a fresh culture medium for further culturing for 7-10 days; and
step 2-4: digesting the cells in the culture flask with a 0.25% trypsin solution, washing the cells sequentially, adding a stem cell culture medium to suspend the cells, inoculating the cells into a culture flask at a density of 5×10⁴ cells/ml for subculture, and subculturing for 2-5 passages for later use, and replacing the stem cell culture medium once per passage during the subculturing.

Preferably, both the DMEM culture medium and the stem cell culture medium contain fetal bovine serum (FBS) with a final concentration of 10% (v/v) and 150 u/mL heparin.

Preferably, the inducing differentiation culture of the stem cells using an active protein in the step 2 or the inducing differentiation culture of the stem cells transfected with the gene of interest using an active protein in the step 4 includes:
adding an active protein into a differentiation culture medium containing stem cells or stem cells transfected with the gene of interest, culturing at 37°C with 5% CO₂, culturing for 3-5 passages for later use, and replacing with the same differentiation culture medium per passage of culture.

Preferably, the differentiation culture medium is a DMEM culture medium.

Preferably, the active protein includes at least one of the following components: TGF-β, platelet-rich plasma (PRP), and SOX9; and further preferably, the active protein includes at least two of the following components: TGF-β, PRP, and SOX9.

Further preferably, the active protein is composed of TGF-β, PRP, and SOX9, and final concentrations of each component in a differentiation culture system are as follows: TGF-β, 4.0-16.0 µg/L; PRP, 0.5-2.0%, v/v; and SOX9, 2.5-10 µg/L; and more preferably, final concentrations of each component of the active protein in the differentiation culture system are as follows: TGF-β, 10 µg/L; PRP, 1%, v/v; and SOX9, 2.5 µg/L.

Preferably, the preparing a stem cell gel solution using the stem cells in the step 3 or the step 5 includes:
mixing stem cells with physiological saline to prepare a cell suspension with a concentration of 0.5×10⁷-1×10⁷ cells/ml, adding sodium hyaluronate to a 1.5%-2.5% (mass concentration) albumin physiological saline solution to prepare a sodium hyaluronate gel solution, and mixing the sodium hyaluronate gel solution with the cell suspension at a volume ratio of 1:0.5-2 to obtain a stem cell gel solution, where the finally obtained stem cell gel solution has a final concentration of 1.5×10⁷-7×10⁷ cells/ml.

Preferably, the constructing a type III collagen 3D scaffold in vitro using the stem cells to finally form tissue-engineered cartilage in the step 3 or the step 5 includes:
preparing a type III collagen 3D scaffold using the differentiated stem cells (2.5×10⁶-5×10⁶ stem cells per scaffold), adding a DMEM cell culture medium containing the active protein to the scaffold, culturing at 37°C with 5% CO₂, replacing the DMEM cell culture medium containing the active protein every 7 days, and continuously culturing for 20-24 days to obtain tissue-engineered cartilage.

Preferably, a method for preparing the type III collagen 3D scaffold includes: mixing a type III collagen solution with a stem cell suspension, adding a fibrinogen solution and thrombin sequentially, mixing uniformly, allowing to stand for 8-12 h, and then culturing at 37°C with 5% CO₂ for 14-21 days to obtain a cell-containing 3D scaffold.

Preferably, final concentrations of components of the active protein in the DMEM cell culture medium are as follows: TGF-β, 4.0-16.0 µg/L; PRP, 0.5-2.0%, v/v; and SOX9, 2.5-10 µg/L; and more preferably, final concentrations of components of the active protein in the DMEM cell culture medium are as follows: TGF-β, 10 µg/L; PRP, 1%, v/v; and SOX9, 2.5 µg/L.

The present invention further provides a stem cell gel solution or tissue-engineered cartilage obtained through the above application.

The present invention also provides an application of the above stem cell gel solution or the above tissue-engineered cartilage in preparing a bone graft.

The present invention solves the problems of insufficient seed cell sources of the stem cell gel solution or tissue-engineered cartilage, inability of in vitro massive expansion, susceptibility to aging after expansion, and has important scientific significance. The obtained stem cell gel solution or tissue-engineered cartilage contains a sufficient number of functionally normal seed cells, and has the function of regulating the proliferation of seed cells and maintaining phenotypic stability thereof.

Furthermore, the present invention solves the problem of integration of the stem cell gel solution or tissue-engineered cartilage graft with host tissue. The active protein expressed by the stem cell gel solution or the tissue-engineered cartilage graft of the present invention is capable of inducing the stem cells of the host tissue to differentiate into chondrocytes, and accelerating the integration of the stem cell gel solution or tissue-engineered cartilage graft with the host tissue. The technology is expected to be further applied in humans, preclinical research is currently underway, and clinical research will commence after obtaining clinical research approval.

### BRIEF DESCRIPTION OF THE DRAWINGS

FIG. 1 illustrates a pCDNA3.1 plasmid and double digestion sites NotI and KpnI in Example 1 of the present invention.
FIG. 2 illustrates a cell morphology of umbilical cord blood-derived mesenchymal stem cells after 7 days of primary culture in Example 1 of the present invention.
FIG. 3 illustrates a cell morphology of umbilical cord blood-derived mesenchymal stem cells after 7 days of first passage culture in Example 1 of the present invention.
FIG. 4 illustrates a cell morphology of umbilical cord blood-derived mesenchymal stem cells after 7 days of second passage culture in Example 1 of the present invention.
FIG. 5 illustrates a cell morphology of umbilical cord blood-derived mesenchymal stem cells after 7 days of third passage culture in Example 1 of the present invention.
FIG. 6 illustrates cell growth after 28 days of continuous screening with a MesenCult MSC Basal Medium stem cell culture medium containing G418 in Example 1 of the present invention, where FIG. 6-1 illustrates cell growth of a BMP7 gene-transfected cell group, and FIG. 6-2 illustrates cell growth of a non-BMP7 gene-transfected cell group.
FIG. 7 illustrates a morphology of a blank 3D scaffold in Example 1 of the present invention.
FIG. 8 illustrates a morphology of BMP7 gene-transfected cartilage without mixed inducing factors in Example 1 of the present invention.
FIG. 9 illustrates a morphology of non-BMP7 gene-transfected cartilage with mixed inducing factors in Example 1 of the present invention.
FIG. 10 illustrates a morphology of BMP7 gene-transfected cartilage with mixed inducing factors in Example 1 of the present invention.
FIG. 11 illustrates a model of rabbit knee joint cartilage defects in Example 2 of the present invention.
FIG. 12 illustrates an effect of repair for 8 weeks in a model group in Example 2 of the present invention.
FIG. 13 illustrates an effect of repair for 8 weeks in a 3D scaffold group in Example 2 of the present invention.
FIG. 14 illustrates an effect of repair for 8 weeks in a BMP7 gene-transfected group in Example 2 of the present invention.
FIG. 15 illustrates an effect of repair for 8 weeks in a BMP7 gene-transfected group with mixed inducing factors in Example 2 of the present invention.
FIG. 16 illustrates a model of rabbit knee joint cartilage defects in Example 3 of the present invention.
FIG. 17 illustrates an effect of repair of tissue-engineered cartilage obtained in Example 3 of the present invention for 8 weeks.
FIG. 18 illustrates a morphology of a blank injection solution in Example 4 of the present invention.
FIG. 19 illustrates a morphology of a stem cell gel solution derived from BMP7 gene transfection without mixed inducing factors in Example 4 of the present invention.
FIG. 20 illustrates a morphology of a stem cell gel solution derived from BMP7 gene transfection with mixed inducing factors in Example 4 of the present invention.
FIG. 21 illustrates a model of rabbit knee joint cartilage defects in Example 4 of the present invention.
FIG. 22 illustrates an effect of repair for 8 weeks in a model group in Example 4 of the present invention.
FIG. 23 illustrates an effect of repair for 8 weeks in a blank gel solution group in Example 4 of the present invention.
FIG. 24 illustrates an effect of repair for 8 weeks in a BMP7 gene-transfected stem cell gel solution group in Example 4 of the present invention.
FIG. 25 illustrates an effect of repair for 8 weeks in a BMP7 gene-transfected stem cell gel solution group with mixed inducing factors in Example 4 of the present invention.
FIG. 26 illustrates a morphology of umbilical cord blood-derived mesenchymal stem cell gel solution with mixed inducing factors in Example 5 of the present invention.
FIG. 27 illustrates a model of rabbit knee joint cartilage defects in Example 5 of the present invention.
FIG. 28 illustrates an effect of repair for 8 weeks in a stem cell gel solution group in Example 5 of the present invention.
FIG. 29 illustrates a model of rabbit knee joint cartilage defects in Example 6 of the present invention.
FIG. 30 illustrates an effect of repair for 8 weeks with a bone marrow-derived mesenchymal stem cell gel solution obtained in Example 6 of the present invention.
FIG. 31 illustrates an effect of repair for 8 weeks with tissue-engineered cartilage constructed from bone marrow-derived mesenchymal stem cells transfected with a gene of interest BMP7 obtained in Example 6 of the present invention.

### DETAILED DESCRIPTION OF EMBODIMENTS

In the examples of the present invention, a pBlue-BMP7 plasmid was purchased from ATCC Company.

In the examples of the present invention, a pcDNA3.1 vector was purchased from Shanghai Boya Biotechnology Co., Ltd.

In the examples of the present invention, a stem cell culture medium is a MesenCult MSC Basal Medium purchased from Shanghai Yes Service Biotech, Inc. The stem cell culture medium is suitable for culturing bone marrow, umbilical cords, umbilical cord blood, placentas, and adipose-derived stem cells. A process of isolating and culturing stem cells derived from umbilical cord blood includes: collecting 2-3 ml of umbilical cord blood from a newborn rabbit, adding a DMEM culture medium, and centrifuging; aspirating an upper fat layer and part of a supernatant from a centrifugate, layering the remaining centrifugate onto a percoll separation solution with a density of 1.073 g/ml, and centrifuging again; isolating intermediate monocytes, washing the cells sequentially, adding a MesenCult MSC Basal Medium stem cell culture medium to suspend the cells, and culturing the cells in an incubator at 37°C with 5% CO₂; discarding non-adherent cells after 3-4 days of culturing, and replacing the MesenCult MSC Basal Medium stem cell culture medium for further culturing, where primary cells cover a bottom of a culture flask after approximately 7-10 days; and subsequently digesting with a 0.25% trypsin solution and continuing subculture with a MesenCult MSC Basal Medium stem cell culture medium. Part of primary umbilical cord blood-derived mesenchymal stem cells begin to adhere to a wall of the culture flask after 12 hours of inoculation, newly adherent cells are round and gradually extend pseudopodia to form cell junctions, and the cells present a spindle-shaped or fusiform appearance like fibroblasts. A cell nucleus is centrally located, and two or more nuclei may be formed. After wall adherence, the cells are amplified in volume, clones are formed with extending protrusions, part of the cells are triangular or polygonal, and the cells have good light reflectivity and pronounced three-dimensionality. After medium change on the 3rd day of culture, the cells are mostly fusiform in morphology, few of the cells are polygonal, clonal cell clusters are formed by cell junctions from pseudopodia, the cells are mostly elongated and fusiform, and distributed radially in concentric circles, without contact inhibition, and the cells are arranged in parallel or grow in a vortex shape. A cell fusion rate may reach more than 95% after 7 days of culture.

A process of isolating and culturing stem cells derived from umbilical cord includes: taking a fresh umbilical cord of a healthy rabbit, washing the umbilical cord with phosphate buffered saline (PBS) and physiological saline sequentially, washing the umbilical cord to remove blood thoroughly, cutting the umbilical cord into small segments of approximately 0.5 cm with a scissor, cutting open each small segment of the umbilical cord, removing two arteries and one vein with the scissor and a forcep, and peeling out Wharton's jelly tissue inside with the forcep; fully mincing the Wharton's jelly tissue of the umbilical cord into pieces of 1 mm×1 mm×1 mm, transferring the minced tissue into a culture flask, spreading the tissue evenly (approximately 2-3 pieces/cm²), and placing the tissue in an incubator at 37°C with 5% CO₂ for inverted culture for approximately 2 h; slowly adding a MesenCult MSC Basal Medium stem cell culture medium along a wall of the culture flask, culturing in an incubator at 37°C with 5% CO₂, replacing the culture medium once every 3 days, and digesting for subculture when the cell fusion rate exceeds 90%:digesting the cells with a 0.25% trypsin solution, washing the digested cells with PBS, and continuing subculture with a MesenCult MSC Basal Medium stem cell culture medium. The culture medium is replaced after 3 days of culturing a primary umbilical cord tissue explant, and part of cells crawl from a periphery of the tissue explant during medium change and look small and fusiform in morphology, and few of the cells are polygonal. After approximately 7 days of culture, the cells begin to proliferate rapidly to form cell colonies of different sizes, newly adherent cells are round and gradually extend pseudopodia to form cell junctions so as to form clonal cell clusters, the cells are mostly elongated and fusiform, and distributed radially in concentric circles, without contact inhibition, and the cells are arranged in parallel or grow in a vortex shape. A cell nucleus is centrally located, two or more nuclei may be formed, and the cells have good light reflectivity and pronounced three-dimensionality. A cell fusion rate may reach more than 90% after approximately 14 days of culture.

A process of isolating and culturing placenta-derived stem cells includes: isolating subamniotic tissue from a rabbit placenta, washing the placenta with PBS and physiological saline sequentially to remove blood in the placenta thoroughly, and then removing arterial and venous blood vessels in the placenta with a surgical scissor and forcep; fully mincing placental tissue into pieces of 1 mm×1 mm×1 mm, transferring the minced tissue into a culture flask, spreading the tissue evenly (approximately 2-3 pieces/cm²), and placing the tissue in an incubator at 37°C with 5% CO₂ for inverted culture for approximately 2 h; slowly adding a MesenCult MSC Basal Medium stem cell culture medium along a wall of the culture flask, culturing in an incubator at 37°C with 5% CO₂, replacing the culture medium once every 3 days, and digesting for subculture when the cell fusion rate exceeds 90%: digesting the cells with a 0.25% trypsin solution, washing the digested cells with PBS, and continuing subculture with a MesenCult MSC Basal Medium stem cell culture medium. After approximately 3 days of culturing a primary placental tissue explant, a small number of cells crawl from a periphery of the tissue explant, and look short, fusiform, and strongly three-dimensional. Subsequently, the cells extend outward slowly, and are mostly elongated and fusiform, and distributed radially in concentric circles, without contact inhibition. The cells are arranged in parallel or grow in a vortex shape and gradually form vortex-like colonies, tissue explants are detached gradually as the number of cells increases, and after approximately 21 days of culture, only a small number of tissue explants remain, and the cell fusion rate may reach approximately 90%.

A process of isolating and culturing adipose-derived stem cells includes: in an animal experiment operating room, anesthetizing a healthy New Zealand rabbit by intraperitoneal injection of an appropriate amount of 10% chloral hydrate, shaving the skin, disinfecting, and cutting an appropriate amount of subcutaneous adipose tissue from a groin thereof under aseptic conditions. Placing the adipose tissue in a beaker in a super clean bench, washing the adipose tissue 3 times with PBS and physiological saline respectively, and removing small blood vessels visible to naked eyes to obtain adipose tissue; cutting the adipose tissue into particles of approximately 1 mm×1 mm×1 mm with a scissor, then adding an equal volume of 0.1% type I collagenase to the adipose tissue, and digesting with shaking at a constant temperature of 37°C for approximately 60 min; then centrifuging at 3500 rpm for 10 min, discarding an upper lipid droplets and a supernatant, resuspending a precipitate with a MesenCult MSC Basal Medium stem cell culture medium, filtering through a 200-mesh sieve, and counting the cells in a cell suspension with a hemocytometer (cell counting plate); inoculating the cells into a culture flask at a density of 2×10⁵ cells/cm², culturing in an incubator at 37°C with 5% CO₂, replacing the culture medium once every 3 days, and digesting for subculture when the cell fusion rate exceeds 90% after 10-14 days of culture; and aspirating a primary cell culture medium, adding a 0.125% trypsin-EDTA (Ethylenediaminetetraacetic acid) digestion solution, washing the digested cells with PBS, and continuing subculture with a MesenCult MSC Basal Medium stem cell culture medium. Primary isolated rabbit adipose-derived mesenchymal stem cells are small spherical cells, and start adherent growth approximately 4 h later after inoculation; and the cells start spreading growth approximately 24 h later after inoculation, look short and fusiform with clear cell outlines and pronounced three-dimensionality, and are arranged in parallel or grow in a vortex shape. The cell fusion rate may reach more than 90% after 7-10 days, the cells proliferate rapidly after subculture and are distributed evenly, presenting a fibroblast-like morphology, and the cell fusion rate may reach 90% for subculture after approximately 6 days.

Furthermore, animal sources of the stem cells used in the present invention are not limited, and for cartilage injury of a certain type of animal, stem cells of the same type of animal are preferably used. For example, experimental animals adopted in the examples of the present invention are rabbits, so stem cell sources are also rabbits.

In the examples of the present invention, a transfection culture medium is OPTI-MEM^{™}I Reduced Serum Medium purchased from Shanghai Mito Bilogical Technology Co., Ltd.

In the examples of the present invention, TGF-β was purchased from Promega Corporation of the United States, platelet-rich plasma (PRP) was purchased from Shanghai Huzheng Biotechnology Co., Ltd., and SOX9 was purchased from Shanghai Huzheng Biotechnology Co., Ltd.

In the examples of the present invention, recombinant type III collagen was purchased from Shanxi Nanba Biotechnology Co., Ltd.

In the examples of the present invention, sodium hyaluronate was purchased from AVT (Shanghai) Pharmaceutical Tech CO. Ltd.

In the examples of the present invention, a method for isolating and in vitro culturing chondrocytes includes: isolating knee joint cartilage from a 2-week-old large-eared white rabbit under aseptic conditions, adding 0.1% type II collagenase for digestion, centrifuging a digestion solution and discarding a supernatant, adding a DMEM culture medium containing 10% (v/v) fetal bovine serum to suspend the cells, inoculating the cells into a culture flask at a density of 5×10⁴ cells/mL for 7 days of culture, and performing subculture for 3 passages (approximately 21 days) for later use when the cell fusion rate reaches 80%-90%. The DMEM culture medium was purchased from Heilongjiang Jiufeng Bioengineering Co., Ltd.

In the examples of the present invention, large-eared white rabbits, newborn rabbits, and New Zealand rabbits were all purchased from the Experimental Animal Center of the Second Affiliated Hospital of Harbin Medical University.

In the description of the present invention, it should be noted that if specific conditions are not specified in the examples, conventional conditions or conditions recommended by the manufacturer shall prevail. If the manufacturers of reagents or instruments used are not specified, they are all conventional products commercially available from the market.

The present invention relates to a method for preparing a stem cell gel solution or constructing tissue-engineered cartilage using transgenic stem cells, where the transgenic stem cells contain genes of interest for inducing the transformation of stem cells into chondrocytes, and the genes of interest may be at least one of BMP7, BMP2, TGF-β, IGF, FGF, and SOX9, preferably BMP7 and/or BMP2. A method for preparing a stem cell gel solution or constructing tissue-engineered cartilage includes the following steps:
step 1: constructing a recombinant plasmid containing a gene of interest;
step 2: isolating and culturing bone marrow, umbilical cords, umbilical cord blood, placentas, or adipose-derived stem cells;
step 3: transfecting the stem cells cultured in the step 2 with the recombinant plasmid constructed in the step 1 to obtain stem cells transfected with the gene of interest;
step 4: inducing differentiation culture of the stem cells transfected with the gene of interest using an active protein; or performing differentiation culture on the stem cells transfected with the gene of interest;
step 5: preparing a stem cell gel solution using the stem cells obtained in the step 4; and
step 6: constructing a recombinant type III collagen 3D culture scaffold in vitro using the stem cells obtained in the step 4 to finally form tissue-engineered cartilage.

The present invention is further described below with reference to the accompanying drawings and specific examples. Those skilled in the art can learn from the content of the present invention and appropriately improve the process parameters for realization. It should be particularly pointed out that all similar substitutions and modifications are obvious to those skilled in the art, and they are deemed to be included in the present invention. The method and application of the present invention have been described through the preferred embodiments, and it is obvious that relevant persons can make changes or appropriate changes and combinations of the methods and applications described herein without departing from the content, spirit and scope of the present invention to achieve and apply the technology of the present invention. Furthermore, the following examples are only intended to illustrate the present invention, and are not intended to limit the present invention.

### Example 1

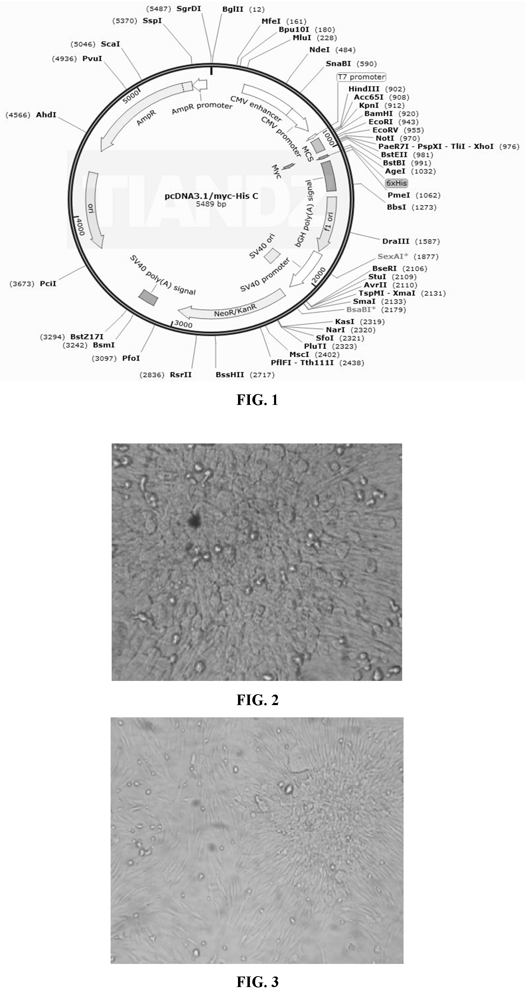

A method is introduced in this example. This method is used to construct tissue-engineered cartilage using transgenic umbilical cord blood-derived mesenchymal stem cells, specifically including the following steps:
step 1: a recombinant plasmid containing a gene of interest BMP7 was constructed; specifically including:
   step 1-1: a pBlue-BMP7 plasmid was double-digested, extracted, and purified; and
   a pBlue-BMP7 prokaryotic expression vector plasmid was double-digested with NotI and KpnI, where a reaction system had a total volume of 20 µl, containing: 5 µl of pBlue-BMP7, 0.5 µl of NotI, 0.5 µl of KpnI, 2 µl of 10×Buffer, and 12 µl of distilled water. Reaction conditions included: incubating was performed in a water bath at 37°C for 2 hours. Agarose gel electrophoresis of 20 µl of an enzyme digestion reaction product was performed to separate DNA fragments. A target DNA fragment with a size of approximately 1.3 Kb was observed and located under an ultraviolet lamp. Gel containing the fragment was carefully cut off with a scalpel, and a DNA fragment of a BMP7 gene was extracted and purified according to the instructions of a gel recovery kit (Shanghai Boya Biotechnology Co., Ltd.). The purified DNA fragment was stored in a refrigerator at -20°C for later use.
   step 1-2: a pcDNA3.1 vector was double-digested, extracted, and purified; and
   a pcDNA3.1 (+) eukaryotic expression vector was double-digested with NotI and KpnI, where a reaction system had a total volume of 20 µl, containing: 5 µl (approximately 500 ng) of pcDNA3.1 (+), 0.5 µl of NotI, 0.5 µl of KpnI, 2 µl of 10×Buffer, and 12 µl of distilled water. Reaction conditions included: incubating was performed in a water bath at 37°C for 2 hours. After the reaction, agarose gel electrophoresis of 20 µl of an enzyme digestion product was performed for separation, and a linearized vector DNA fragment with a size of approximately 5.4 Kb was observed and located under an ultraviolet lamp (enzyme digestion sites are at 912-970, as shown in FIG. 1. Gel containing the fragment was cut off with a scalpel, a pcDNA3.1 vector DNA fragment was extracted and purified according to the instructions of a gel extraction and purification kit (Shanghai Boya Biotechnology Co., Ltd.), and the purified vector fragment was stored in a refrigerator at -20°C for later use.
   step 1-3: the BMP7 gene fragment purified in the step 1-1 and the pcDNA3.1 vector fragment purified in the step 1-2 were ligated under the action of T₄DNA ligase, and a reaction system contained: 6 µl of BMP7, 2 µl of pcDNA3.1, 1 µl of 10×Buffer, 0.5 µl of T₄DNA ligase, 0.5 µl of distilled water; and the reaction system had a total volume of 10 µl. Reaction conditions included: ligating was performed in a water bath at 6°C for 8 hours. A ligation product (i.e., a pcDNA3.1-BMP7 vector plasmid) was stored in a refrigerator at -20°C for later use.
   Step 1-4: the pcDNA3.1-BMP7 plasmid constructed in the step 1-3 was transformed into DH5α competent cells. Then amplification culture was performed on plasmid-containing cells. The pcDNA3.1-BMP7 plasmid was extracted and purified from the amplified cells according to the instructions of a plasmid extraction kit (Shanghai Boya Biotechnology Co., Ltd.). The extracted plasmid was sequenced to confirm the correctness of a BMP7 gene sequence. Sequencing method: taking a pcDNA3.1-BMP7 eukaryotic expression vector plasmid as a template, 2 PCR amplification reactions were performed using T3 (5'-TAA TAC GAC TCA CTA TAG GG-3', SEQ ID NO.1) and T7 (5'-ATT AAC CCT CAC TAA AG-3', SEQ ID NO.2) sequencing primers, reaction products were sequenced, and sequencing results of the above two reactions were spliced with computer software (determined by Shanghai Boya Biotechnology Co., Ltd.). The results showed that a DNA sequence obtained by splicing was completely consistent with a full-length sequence of the BMP7 gene reported by National Center for Biotechnology Information (GenBank) without mutation.
Step 2: stem cells were isolated and cultured; the following steps were specifically included:
   step 2-1: umbilical cord blood was collected immediately after birth of a newborn rabbit, the umbilical cord blood was placed in a sterile tube containing a 3.8% sodium citrate anticoagulant, and 2-3 ml of umbilical cord blood was transferred into a 50 ml sterile centrifuge tube containing 10 ml of a DMEM culture medium (note: the DMEM culture medium further contained 10% (volume percentage) FBS and 150 u/ml heparin); and the centrifuge tube was centrifuged at a centrifugal force of 300 g for 5 min;
   step 2-2: after centrifugation, an upper fat layer and part of a supernatant were aspirated from the centrifuge tube, remaining liquid was layered onto a percoll separation solution with a density of 1.073 g/ml at a volume ratio of 1:2, and a resulting mixture was centrifuged at a centrifugal force of 900 g for 30 min; and
   step 2-3: after intermediate monocytes were isolated, isolated cells were washed for multiple times. Then a MesenCult MSC Basal Medium stem cell culture medium was added to suspend the cells, the cells were inoculated in a culture flask at a cell density of 5×10⁶ cells/ml with an inoculation volume of 3 ml, and the cells were cultured in an incubator at 37°C with 5% CO₂. The cells were washed 3 times with a PBS solution after 3-4 days of culture, non-adherent cells were discarded, the MesenCult MSC Basal Medium stem cell culture medium was replaced for further culture, and primary cells covered a bottom of a culture flask after approximately 7 days, with results shown in FIG. 2.
   Note: The above MesenCult MSC Basal Medium stem cell culture medium further contained fetal bovine serum (FBS) with a final concentration of 10% (volume percentage) and heparin with a final concentration of 150 u/mL respectively.
   Step 2-4: when the primary cells (P0) covered the bottom of a culture flask, the cells were digested with a 0.25% trypsin solution. The digested cells were inoculated in a MesenCult MSC Basal Medium stem cell culture medium at a density of 5×10⁴ cells/ml (the cells inoculated this time belonged to the first passage (P1)), subculture was continued, subculturing was performed once every approximately 7 days, and cells of the second passage (P2) and the third passage (P3) were obtained sequentially.

Cell morphology observation:
Initial inoculation stage: Part of primary umbilical cord blood-derived mesenchymal stem cells (P0) began to adhere to a wall of the culture flask after 12 hours of inoculation, newly adherent cells were round and gradually extended pseudopodia to form junctions with adjacent cells, and the cells presented a spindle-shaped or fusiform appearance like fibroblasts. A cell nucleus was centrally located, and two or more nuclei might be formed.

Growth stage: After wall adherence, the cells were amplified in volume, cell clones were formed with more extending protrusions, and part of the cells were triangular or polygonal. The cells had good light reflectivity and pronounced three-dimensionality.

After medium change (3 days later), the cells were mostly fusiform, few of the cells were polygonal, cell junctions were achieved by pseudopodia, clonal cell clusters were formed, the cells were mostly elongated and fusiform, and distributed radially in concentric circles, without contact inhibition between the cells, and the cells were arranged in parallel or grew in a vortex shape.

Harvest stage: A cell fusion rate may reach more than 95% after 7 days of culture.

Morphological changes after subculture: P1 cells still formed cell colonies similar to primary cells, P2 cells formed fewer colonies, and the cells no longer formed colonies from P3. With the increase of passages, cell adherence capability and adaptability were enhanced, the cells adhered and proliferated rapidly after subculture, and the cells were flattened and had weak light reflectivity and three-dimensionality, with the results shown in FIGS. 3 to 5.

Step 3: the stem cells cultured in the step 2 were transfected with the recombinant plasmid constructed in the step 1 to obtain stem cells transfected with the gene of interest (hereinafter referred to as "a BMP7 gene-transfected cell group"); and a non-BMP7 gene-transfected cell group was set up simultaneously, with specific operation steps as follows:
I. Operation steps of the BMP7 gene-transfected cell group:
   a) Preparation of a DNA-liposome complex: Two sterile test tubes were taken and marked as A and B. Tube A: 250 µl of a transfection culture medium and 1.5 µl of a pcDNA3.1-BMP7 plasmid were added and mixed uniformly; and Tube B: 250 µl of a transfection culture medium and 10 µl of a Lipofectamine^{™} 2000 liposome (purchased from Gibco) and mixed uniformly. A resulting mixture was allowed to stand for 5 minutes.
      Formation of a DNA-liposome complex: Solutions in Tube A and Tube B were combined and mixed gently, and a resulting mixture was allowed to stand at room temperature for 20 min to form the DNA-liposome complex.
   b) Cell transfection: A six-well plate with P3 stem cells in an exponential growth phase cultured therein was taken. A MesenCult MSC Basal Medium in the wells was aspirated, 500 µl of a serum-free high-glucose DMEM culture medium and 500 µl of the DNA-liposome complex were added to each well, and the six-well plate was mixed gently, and placed in an incubator at 37°C with 5% CO₂ for culture.
   c) Medium change and screening: After 6 hours of culture, the serum-free culture medium containing the DNA-liposome complex in the wells was aspirated. 2 ml of the MesenCult MSC Basal Medium stem cell culture medium was added to each well for further culture. After 24 hours of transfection, the MesenCult MSC Basal Medium stem cell culture medium containing 350 µg/ml G418 was replaced for screening stably transfected cells, then a screening solution was replaced once every 4 days, and cell activity and growth state were observed under a microscope.
   d) An appropriate amount of a culture supernatant was collected on the 7th, 14th and 28th days after screening respectively to detect an expression level of BMP7 protein.
II. Operation steps of the non-BMP7 gene-transfected cell group: The operation steps for this cell group were basically the same as those of the "BMP7 gene-transfected cell group", and the only difference lied in the substance added to Tube A in the step 1: when preparing the "DNA-liposome complex", 250 µl of a transfection culture medium and 1.5 µl of a pcDNA3.1 plasmid (containing no BMP7 gene) were added to Tube A and mixed uniformly; and all other steps, including culture, screening, medium change, and detection time points (the supernatant was collected on the 7th, 14th and 28th days), were the same as those of the "BMP7 gene-transfected cell group".

To ensure data reliability, 5 parallel samples were set for all test groups. A mean of measured values of the 5 parallel samples was calculated as a BMP7 expression level.

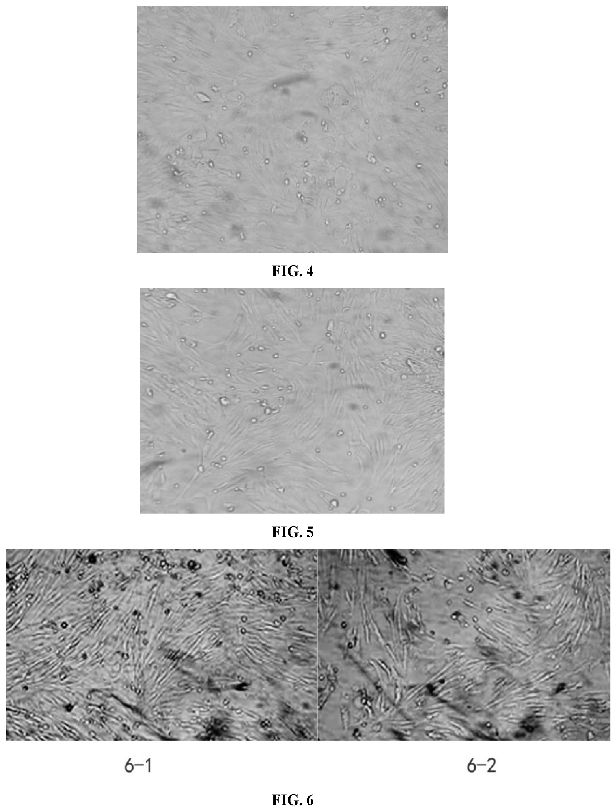

The BMP7 expression was detected by enzyme linked immunosorbent assay (ELISA) on the 7th, 14th and 28th days of culture. The results showed that some cells in both the BMP7 gene-transfected cell group and the non-BMP7 gene-transfected cell group still survived and grew within 28 days of continuous screening with G418 (Geneticin) (see FIGS. 6-1 and 6-2), and the surviving cells were the cells successfully transfected with the pcDNA3.1-BMP7 plasmid. Compared with the non-BMP7 gene-transfected cell group, the BMP7 protein expression level of the BMP7 gene-transfected cells was significantly improved (*p<0.05*) (specific data are shown in Table 1). Conclusion: This indicates that the BMP7 gene had been successfully integrated into a cell genome to achieve stable transfection.

**Table 1 Mean BMP7 expression (pg/ml, n=5)**

| Group | 7 days | 14 days | 28 days |
|---|---|---|---|
| Non-BMP7 gene-transfected cell group | 30.12 | 29.86 | 31.54 |
| BMP7 gene-transfected cell group | 102.67 | 135.46 | 158.98 |

Step 4: inducing differentiation culture of the stem cells transfected with the gene of interest using an active protein; and
this step is intended to explore effects of different active proteins and combinations thereof on inducing the differentiation of BMP7 gene-transfected stem cells into chondrocytes, and a specific experimental process is as follows:
(1) testing an induction effect of single active protein: in this experiment, the induction effects of three active proteins (TGF-β, PRP (PRP), and SOX9) respectively on the BMP7 gene-transfected stem cells were tested when the three active proteins were used alone.

A specific method is as follows:
1) Cell preparation: A 96-well culture plate was taken, 100 µl of a cell suspension (the cell suspension contained 1×10⁵ cells/ml of stem cells transfected with the pcDNA3.1-BMP7 plasmid) was added to each well, and the culture plate was placed in an incubator at 37°C with 5% CO₂ for 24 hours of culture.
2) Culture: After 24 hours of culture, the culture medium was discarded. Then a new culture medium diluted with the DMEM culture medium and containing different concentrations of active proteins was added respectively, with details specifically including:
   TGF-β group: concentrations were 4 µg/L, 10 µg/L, and 16 µg/L respectively;
   PRP group: concentrations were 0.5%, 1.0%, and 2.0% (volume percentage) respectively;
   SOX9 group: concentrations were 2.5 µg/L, 5 µg/L, and 10 µg/L respectively; and
   3 replicate wells were set for each experimental group of each concentration.
   Setting up control groups (3 replicate wells were set for each control group):
      chondrocyte control group: normal chondrocytes were added, where isolation and in vitro culture of chondrocytes are detailed above (without BMP7 gene transfection and without inducing factors, only a DMEM culture medium was added);
      stem cell control group: non-transfected normal stem cells were added, and only the DMEM culture medium (i.e., the stem cells obtained in the step 2, without BMP7 gene transfection and without inducing factors, and only a DMEM culture medium was added) was added;
      BMP7 gene-transfected stem cell-containing control group: BMP7 gene-transfected stem cells were added, and only a DMEM culture medium (i.e., an inducible protein was not added) was added;
      non-BMP7 gene-transfected stem cell + active protein group: non-transfected normal stem cells were added, but a DMEM culture medium containing an active protein (i.e., the stem cells obtained in the step 2, without BMP7 gene transfection, and only a DMEM culture medium containing an inducible protein was added) was added; and
      blank control group: no cells were added, and only a DMEM culture medium containing an active protein was added.
3) Detection: all culture wells were cultured in an incubator, the culture medium was collected on the 7th day (P1), 14th day (P2), and 21st day (P3) of culture respectively, and contents of type II collagen and glycosaminoglycans therein were detected by ELISA (conventional indicators for detecting chondrocyte differentiation).

The experimental results are shown in Tables 2 and 3, indicating that:
a) In the TGF-β group, the PRP group, and the SOX9 group, all the BMP7 gene-transfected stem cells secreted more type II collagen and glycosaminoglycans, and secretion amounts were significantly higher than those of the "non-BMP7 gene-transfected stem cell + active protein group", the "stem cell control group", and the "BMP7 gene-transfected stem cell-containing control group". Effects of TGF-β and SOX9 reached a plateau at medium concentrations (10 µg/L and 5 µg/L) (i.e., the effect was no longer enhanced significantly after concentration increase), while the effect of PRP was enhanced with the concentration increase, and the effect was the strongest at a high concentration (2%);
b) the secretion amounts of type II collagen and glycosaminoglycans in the "non-BMP7 gene-transfected stem cell + active protein group" were also significantly higher than those of the "BMP7 gene-transfected stem cell-containing control group" and the "stem cell control group";
c) the secretion amount of the "BMP7 gene-transfected stem cell-containing control group" was also significantly higher than that of the "stem cell control group"; and
d) secretion levels of all experimental groups were lower than that of the "chondrocyte control group".

Seen from the above results, the BMP7 protein expressed by BMP7 gene-transfected stem cells induced the differentiation of stem cells into chondrocytes, and on this basis, any one of the inducing factors TGF-β, PRP, and SOX9 was added to induce the differentiation of stem cells into chondrocytes. Under conditions of this experiment, optimal induction concentrations of TGF-β, PRP, and SOX9 were 10.0 µg/L, 2%, and 5.0 µg/L respectively.

**Table 2 Mean type II collagen secretion of each group (ng/ml)**

| Group | Final concentration of inducing factor | Detection time (day) | | |
|---|---|---|---|---|
| | | 7 | 14 | 21 |
| Blank control group | - | 0 | 0 | 0 |
| Chondrocyte control group | - | 923.3 | 978.4 | 965.3 |
| Stem cell control group | - | 220.8 | 234.0 | 231.2 |
| BMP7 gene-transfected stem cell-containing control group | - | 496.3 | 523.5 | 524.7 |
| TGF-β₁ group of BMP7 gene-transfected stem cells | 4.0 µg/L | 504.2 | 531.6 | 523.3 |
| | 10.0 µg/L | 521.5 | 582.0 | 551.3 |
| | 16.0 µg/L | 523.4 | 579.5 | 564.3 |
| TGF-β₁ group of non-BMP7 gene-transfected stem cells | 4.0 µg/L | 490.5 | 526.7 | 520.4 |
| | 10.0 µg/L | 506.7 | 561.5 | 541.6 |
| | 16.0 µg/L | 513.3 | 568.3 | 544.0 |
| PRP group of BMP7 gene-transfected stem cells | 0.5% | 512.0 | 537.6 | 529.6 |
| | 1.0% | 540.8 | 578.6 | 561.0 |
| | 2.0% | 581.3 | 598.4 | 590.6 |
| PRP group of Non-BMP7 gene-transfected stem cells | 0.5% | 510.2 | 530.5 | 520.4 |
| | 1.0% | 530.4 | 563.1 | 542.2 |
| | 2.0% | 561.8 | 576.7 | 571.3 |
| SOX9 group of BMP7 gene-transfected stem cells | 2.5 µg/L | 501.0 | 537.6 | 529.6 |
| | 5.0 µg/L | 578.0 | 612.4 | 601.6 |
| | 10.0 µg/L | 572.3 | 620.3 | 599.2 |
| SOX9 group of non-BMP7 gene-transfected stem cells | 2.5 µg/L | 498.5 | 521.1 | 518.6 |
| | 5.0 µg/L | 561.4 | 586.3 | 577.9 |
| | 10.0 µg/L | 564.0 | 585.7 | 571.4 |

**Table 3 Mean glycosaminoglycan secretion level of each group (µg/ml)**

| Group | Final concentration of inducing factor | Detection time (day) | | |
|---|---|---|---|---|
| | | 7 | 14 | 21 |
| Blank control group | - | 0 | 0 | 0 |
| Chondrocyte control group | - | 19.20 | 34.19 | 29.56 |
| Stem cell control group | - | 1.45 | 2.56 | 2.48 |
| BMP7 gene-transfected stem cell-containing control group | - | 2.32 | 8.82 | 8.27 |
| TGF-β₁ group of BMP7 gene-transfected stem cells | 4.0 µg/L | 3.51 | 10.90 | 9.26 |
| | 10.0 µg/L | 10.22 | 15.23 | 14.66 |
| | 16.0 µg/L | 11.02 | 14.66 | 13.86 |
| TGF-β₁ group of non-BMP7 gene-transfected stem cells | 4.0 µg/L | 3.01 | 9.45 | 9.10 |
| | 10.0 µg/L | 8.21 | 12.81 | 11.97 |
| | 16.0 µg/L | 8.13 | 12.54 | 12.02 |
| PRP group of BMP7 gene-transfected stem cells | 0.5% | 3.63 | 12.87 | 11.38 |
| | 1.0% | 5.07 | 12.58 | 11.24 |
| | 2.0% | 11.63 | 16.69 | 14.17 |
| PRP group of Non-BMP7 gene-transfected stem cells | 0.5% | 3.24 | 9.72 | 10.03 |
| | 1.0% | 4.54 | 10.49 | 10.44 |
| | 2.0% | 8.03 | 12.71 | 12.03 |
| SOX9 group of BMP7 gene-transfected stem cells | 2.5 µg/L | 4.31 | 13.37 | 11.25 |
| | 5.0 µg/L | 12.01 | 17.85 | 16.42 |
| | 10.0 µg/L | 11.63 | 16.96 | 15.71 |
| SOX9 group of non-BMP7 gene-transfected stem cells | 2.5 µg/L | 3.62 | 9.36 | 9.57 |
| | 5.0 µg/L | 9.81 | 15.87 | 14.73 |
| | 10.0 µg/L | 9.74 | 15.31 | 14.00 |

(2) Testing the effect of inducing with two active proteins in combination: On the basis of experiment (1) above, the effect of inducing with both two active proteins was further tested. Three combinations of TGF-β+PRP, TGF-β+SOX9, and PRP+SOX9 were tested respectively.

A specific method is as follows:
1) A 96-well culture plate was taken, 100 µl of a BMP7 gene-transfected stem cell suspension (1×10⁵ cells/ml) was added to each well, and cells were cultured for 24 hours.
2) Addition of mixed inducing factors: After 24 hours of culture, the culture medium was discarded. Mixed culture media of different concentration combinations diluted with DMEM were added respectively.

A mixed culture medium of TGF-β and PRP had 3 different concentration combinations, specifically including: a) The mixed culture medium contained 10.0 µg/L TGF-β and 2% PRP; b) the mixed culture medium contained 10.0 µg/L TGF-β and 1% PRP; c) the mixed culture medium contained 4.0 µg/L TGF-β (since there was no difference between a medium-dose group and a high-dose group, with the effect of the medium-dose group reaching a plateau, a concentration lower than that of the medium-dose group was selected for screening, the same applies hereinafter) and 2% PRP.

A mixed culture medium of TGF-β and SOX9 had 3 different concentration combinations, specifically including: a) the mixed culture medium contained 10.0 µg/L TGF-β and 5.0 µg/L SOX9; b) the mixed culture medium contained 10.0 µg/L TGF-β and 2.5 µg/L SOX9; and c) the mixed culture medium contained 4.0 µg/L TGF-β and 5.0 µg/L SOX9.

A mixed culture medium of PRP and SOX9 had 3 different concentration combinations, specifically including: a) the mixed culture medium contained 2% PRP and 5.0 µg/L SOX9; b) the mixed culture medium contained 2% PRP and 2.5 µg/L SOX9; and c) the mixed culture medium contained 1% PRP and 5.0 µg/L SOX9. Three replicate wells were set for each combination of each mixed culture medium.

3) Setting of control groups: The control groups in Experiment (1) were also set up (a chondrocyte control group, a stem cell control group, a BMP7 gene-transfected stem cell control group, a non-transfected stem cell + active protein control group, and a blank control group).

4) Detection: Similarly, the culture medium was collected on the 7th, 14th and 21st days of culture, and contents of type II collagen and glycosaminoglycans were detected by ELISA.

The results were shown in Table 4 and Table 5, indicating that:
a) in a TGF-β+PRP group, a TGF-β+SOX9 group, and a PRP+SOX9 group, secretion amounts of type II collagen and glycosaminoglycans by BMP7 gene-transfected stem cells further increased, which were significantly higher than those of a non-BMP7 gene-transfected stem cell + active protein control group, a stem cell group, and a BMP7 gene-transfected stem cell group; the effects of the mixed culture media composed of TGF-β (10.0 µg/L) + PRP (1%) and TGF-β (10.0 µg/L) + SOX9 (2.5 µg/L) reached a plateau, while the effect of PRP (2%) + SOX9 (5.0 µg/L) was the strongest;
b) the secretion amounts of type II collagen and glycosaminoglycans in the non-BMP7 gene-transfected stem cell + active protein control group were significantly higher than those of the BMP7 gene-transfected stem cell-containing control group and the stem cell control group. The effects of the mixed culture media composed of TGF-β (10.0 µg/L) + PRP (1%) and TGF-β (10.0 µg/L) + SOX9 (2.5 µg/L) reached a plateau, while the effect of PRP (2%) + SOX9 (5.0 µg/L) was the strongest;
c) the secretion amounts of type II collagen and glycosaminoglycans in the BMP7 gene-transfected stem cell control group were significantly increased, which were significantly higher than those of the stem cell control group; and
d) secretion levels of type II collagen and glycosaminoglycans in the stem cell control group, the BMP7 gene-transfected stem cell control group, the non-BMP7 gene-transfected stem cell + active protein control group, and the BMP7 gene-transfected stem cell + active protein group were all lower than that of the chondrocyte group.

Seen from the above results, the BMP7 protein expressed by BMP7 gene-transfected stem cells induced the differentiation of stem cells into chondrocytes, and on this basis, added mixed inducing factors including TGF-β+PRP, TGF-β+SOX9, and PRP+SOX9 all induced the differentiation of stem cells into chondrocytes. That is, on the basis of BMP7 gene transfection, the combination of two active proteins induced the differentiation of stem cells into chondrocytes more effectively than the use of one active protein. Optimal induction concentrations of a TGF-β and PRP combination were 10.0 µg/L and 1% respectively; optimal induction concentrations of a TGF-β and SOX9 combination were 10.0 µg/L and 2.5 µg/L respectively; and optimal induction concentrations of a PRP and SOX9 combination were 2% and 5.0 µg/L respectively.

**Table 4 Mean type II collagen secretion of each group (ng/ml)**

| Group | Final concentration of inducing factor | Detection time (day) | | |
|---|---|---|---|---|
| | | 7 | 14 | 21 |
| Blank control | - | 0 | 0 | 0 |
| Chondrocyte | - | 896.1 | 1009. 3 | 989.1 |
| Stem cells | - | 211.7 | 234.4 | 225.0 |
| BMP7 gene-transfected stem cell-containing | - | 506.3 | 513.5 | 510.4 |
| BMP7 gene-transfected stem cells TGF-β1+PRP | 4.0 µg/L+2% | 621.7 | 683.4 | 651.2 |
| | 10.0 µg/L+1% | 646.2 | 723.1 | 702.5 |
| | 10.0 µg/L+2% | 651.9 | 738.8 | 699.0 |
| Non-BMP7 gene-transfected stem cells TGF-β1+PRP | 4.0 µg/L+2% | 553.2 | 580.4 | 572.2 |
| | 10.0 µg/L+1% | 621.4 | 675.6 | 651.7 |
| | 10.0 µg/L+2% | 623.7 | 670.3 | 654.5 |
| BMP7 gene-transfected stem cells TGF-β1+SOX9 | 4.0 µg/L+5.0 µg/L | 635.3 | 745.0 | 700.5 |
| | 10.0 µg/L+2.5 µg/L | 685.2 | 756.0 | 714.1 |
| | 10.0 µg/L+5.0 µg/L | 678.9 | 761.7 | 709.6 |
| Non-BMP7 gene-transfected stem cells TGF-β1+SOX9 | 4.0 µg/L+5.0 µg/L | 543.2 | 562.6 | 567.2 |
| | 10.0 µg/L+2.5 µg/L | 630.4 | 643.7 | 655.9 |
| | 10.0 µg/L+5.0 µg/L | 628.7 | 645.0 | 650.1 |
| BMP7 gene-transfected stem cells PRP+SOX9 | 1%+5.0 µg/L | 513.9 | 546.4 | 541.0 |
| | 2%+2.5 µg/L | 615.7 | 687.9 | 650.3 |
| | 2%+5.0 µg/L | 699.0 | 745.2 | 712.6 |
| Non-BMP7 gene-transfected stem cells PRP+SOX9 | 1%+5.0 µg/L | 510.5 | 532.1 | 530.6 |
| | 2%+2.5 µg/L | 587.4 | 607.8 | 612.3 |
| | 2%+5.0 µg/L | 628.5 | 687.9 | 681.4 |

**Table 5 Mean glycosaminoglycan secretion level of each group (µg/ml)**

| Group | Final concentration of inducing factor | Detection time (day) | | |
|---|---|---|---|---|
| | | 7 | 14 | 21 |
| Blank control | - | 0 | 0 | 0 |
| Chondrocyte | - | 20.61 | 36.38 | 31.73 |
| Stem cells | - | 1.15 | 3.41 | 3.12 |
| BMP7 gene-transfected stem cell-containing control group | - | 3.02 | 9.64 | 9.72 |
| BMP7 gene-transfected stem cells TGF-β1+PRP | 4.0 µg/L+2% | 4.09 | 10.19 | 9.67 |
| | 10.0 µg/L+1% | 14.65 | 20.93 | 18.65 |
| | 10.0 µg/L+2% | 15.19 | 19.95 | 17.92 |
| Non-BMP7 gene-transfected stem cells TGF-β1+PRP | 4.0 µg/L+2% | 3.56 | 9.87 | 9.20 |
| | 10.0 µg/L+1% | 10.23 | 16.12 | 15.13 |
| | 10.0 µg/L+2% | 10.78 | 16.75 | 15.78 |
| BMP7 gene-transfected stem cells TGF-β1+SOX9 | 4.0 µg/L+5.0 µg/L | 5.35 | 16.22 | 15.49 |
| | 10.0 µg/L+2.5 µg/L | 16.18 | 23.85 | 21.04 |
| | 10.0 µg/L+5.0 µg/L | 15.90 | 22.08 | 20.61 |
| Non-BMP7 gene-transfected stem cells TGF-β1+SOX9 | 4.0 µg/L+5.0 µg/L | 4.12 | 13.09 | 13.11 |
| | 10.0 µg/L+2.5 µg/L | 13.27 | 19.45 | 18.84 |
| | 10.0 µg/L+5.0 µg/L | 13.18 | 19.72 | 17.28 |
| BMP7 gene-transfected stem cells PRP+SOX9 | 1%+5.0 µg/L | 5.90 | 14.27 | 14.18 |
| | 2%+2.5 µg/L | 11.34 | 20.21 | 19.43 |
| | 2%+5.0 µg/L | 15.43 | 25.87 | 24.76 |
| Non-BMP7 gene-transfected stem cells PRP+SOX9 | 1%+5.0 µg/L | 4.46 | 15.37 | 15.90 |
| | 2%+2.5 µg/L | 9.96 | 18.43 | 17.47 |
| | 2%+5.0 µg/L | 12.78 | 22.96 | 21.38 |

(3) Testing the effect of inducing with three active proteins in combination: On the basis of experiment (2) above, the effect of inducing with all three active proteins (TGF-β, PRP, and SOX9) was further investigated. A specific method is as follows:
1) The same steps in Experiment (1) and Experiment (2) were adopted.
2) After 24 hours of culture, the culture medium was discarded. Mixed culture media of three different protein concentration combinations diluted with DMEM were added respectively. The mixed culture media specifically included:
   a) The mixed culture medium contained 10.0 µg/L TGF-β, 2% PRP, and 5.0 µg/L SOX9;
   b) the mixed culture medium contained 10.0 µg/L TGF-β, 1% PRP, and 2.5 µg/L SOX9; and
   c) the mixed culture medium contained 4.0 µg/L TGF-β, 2% PRP, and 5.0 µg/L SOX9.
   Three replicate wells were set for each combination of each mixed culture medium.
3) Setting of control groups: exactly the same as those in Experiment (1) and Experiment (2).
4) Detection: On the 7th, 14th and 21st days of culture, contents of type II collagen and glycosaminoglycans were detected by ELISA.

The results were shown in Table 6 and Table 7, indicating that:
a) In a mixed culture group of TGF-β+PRP+SOX9, secretion amounts of type II collagen and glycosaminoglycans by BMP7 gene-transfected stem cells significantly increased, which were significantly higher than those of a non-BMP7 gene-transfected stem cell + active protein control group, a stem cell group, and a BMP7 gene-transfected stem cell group. a) The effect of a mixed culture medium of TGF-β (10.0 µg/L) + PRP (1%) + SOX9 (2.5 µg/L) reached a plateau, the secretion level thereof was equivalent to the secretion level of chondrocytes, and the mixed culture medium had an optimal induction combination; and
b) the secretion amounts of type II collagen and glycosaminoglycans in the non-BMP7 gene-transfected stem cell + active protein control group were significantly higher than those of the BMP7 gene-transfected stem cell-containing control group and the stem cell control group. The effect of a mixed culture medium of TGF-β (10.0 µg/L) + PRP (1%) + SOX9 (2.5 µg/L) reached a plateau, the secretion level thereof was slightly lower than the secretion level of chondrocytes, and the mixed culture medium had an optimal induction combination; and
c) the secretion amount of the "BMP7 gene-transfected stem cell-containing control group was significantly higher than that of the stem cell group; and
d) secretion levels of type II collagen and glycosaminoglycans in the stem cell control group, the BMP7 gene-transfected stem cell control group, the non-BMP7 gene-transfected stem cell + active protein control group, and the BMP7 gene-transfected stem cell + active protein group (TGF-β (4.0 µg/L) + PRP (2%) + SOX9 (5.0 µg/L)) were all lower than that of the chondrocyte group.

Seen from the above results, the BMP7 protein expressed by BMP7 gene-transfected stem cells induced the differentiation of stem cells into chondrocytes, and on this basis, exogenous inducing factors TGF-β, PRP, and SOX9 were added to synergistically and significantly induce the differentiation of stem cells into chondrocytes. In an experimental system of the present invention, optimal induction concentrations of the TGF-β+PRP+SOX9 combination were 10.0 µg/L, 1%, and 2.5 µg/L respectively.

**Table 6 Mean type II collagen secretion of each group (ng/ml)**

| Group | Final concentration of inducing factor | Detection time (day) | | |
|---|---|---|---|---|
| | | 7 | 14 | 21 |
| Blank control | - | 0 | 0 | 0 |
| Chondrocyte | - | 876.2 | 956.2 | 914.1 |
| Stem cells | - | 201.4 | 220.6 | 213.8 |
| BMP7 gene-transfected stem cell-containing control group | - | 475.2 | 516.0 | 504.8 |
| BMP7 gene-transfected stem cell TGF-β1+PRP+SOX9 | 4.0 µg/L+2%+5.0 µg/L | 754.3 | 802.4 | 799.1 |
| | 10.0 µg/L+1%+2.5 µg/L | 900.5 | 961.2 | 906.7 |
| | 10.0 µg/L+2%+5.0 µg/L | 892.4 | 968.7 | 910.3 |
| Non-BMP7 gene-transfected stem cell TGF-β1+PRP+SOX9 | 4.0 µg/L+2%+5.0 µg/L | 682.7 | 754.2 | 748.0 |
| | 10.0 µg/L+1%+2.5 µg/L | 824.9 | 904.8 | 856.7 |
| | 10.0 µg/L+2%+5.0 µg/L | 825.6 | 912.1 | 860.2 |

**Table 7 Mean glycosaminoglycan secretion level of each group (µg/ml)**

| Group | Final concentration of inducing factor | Detection time (day) | | |
|---|---|---|---|---|
| | | 7 | 14 | 21 |
| Blank control | - | 0 | 0 | 0 |
| Chondrocyte | - | 23.43 | 40.57 | 35.21 |
| Stem cells | - | 2.24 | 3.79 | 3.19 |
| BMP7 gene-transfected stem cell-containing control group | - | 4.01 | 10.15 | 9.22 |
| BMP7 gene-transfected stem cell TGF-β1+PRP+SOX9 | 4.0 µg/L+2%+5.0 µg/L | 15.80 | 30.47 | 25.05 |
| | 10.0 µg/L+1%+2.5 µg/L | 24.78 | 41.54 | 36.71 |
| | 10.0 µg/L+2%+5.0 µg/L | 23.03 | 40.74 | 34.89 |
| Non-BMP7 gene-transfected stem cell TGF-β1+PRP+SOX9 | 4.0 µg/L+2%+5.0 µg/L | 10.24 | 25.77 | 20.27 |
| | 10.0 µg/L+1%+2.5 µg/L | 19.83 | 35.89 | 27.45 |
| | 10.0 µg/L+2%+5.0 µg/L | 18.67 | 34.25 | 27.64 |

Therefore, in a subsequent experiment of the present invention, final component concentrations of active proteins used in a differentiation culture system were as follows: TGF-β, 10 µg/L; PRP, 1%, v/v; and SOX9, 2.5 µg/L.

Step 5: The stem cells induced in the step 4 (the above optimal concentration combination was selected: TGF-β 10.0 µg/L, PRP 1%, and SOX9 2.5 µg/L) were used to construct a recombinant type III collagen 3D culture scaffold in vitro to finally form tissue-engineered cartilage. The following groups were set up for the experiment:
a) A gene of interest-transfected + inducing factor group: BMP7 gene-transfected stem cells induced by an active protein were used.
b) A non-gene of interest-transfected + inducing factor group: Non-BMP7 gene-transfected stem cells induced by an active protein were used.
c) A gene of interest-transfected group: BMP7 gene-transfected stem cells not induced by an active protein were used.
d) A blank recombinant type III collagen 3D scaffold group: a blank scaffold containing no cells.

Operation for the gene of interest-transfected + inducing factor group was as follows: The transgenic stem cell suspension with a concentration of 1×10⁷ cells/ml obtained in the step 4 was taken to construct a recombinant type III collagen 3D scaffold containing approximately 2×10⁶ cells per scaffold according to the "3D Scaffold Preparation Method" described below, and this operation was performed in a 12-well culture plate. The 12-well plate was placed in an incubator at 37°C with 5% CO₂ for 12 hours of culture, such that the cells were evenly distributed in a scaffold space and fully combined with a scaffold material. After 12 hours of culture, the recombinant type III collagen 3D scaffold containing stem cells was transferred from the 12-well culture plate to a 6-well culture plate, 3 ml of a DMEM culture medium containing mixed active proteins (final concentrations: TGF-β1 (10.0 µg/L) + PRP (1%) + SOX9 (2.5 µg/L)) and 10% fetal bovine serum was added to each well, the scaffold was cultured in an incubator at 37°C with 5% CO₂, the culture medium was replaced every 3 days, and morphological observation was performed on the 0th, 7th and 14th days of culture respectively. Additionally, the culture medium was collected, and the secretion amounts of type II collagen and glycosaminoglycans were determined by ELISA.

The non-gene of interest-transfected + inducing factor group: All operations for this group were exactly the same as those of the "gene of interest-transfected + inducing factor group". The only difference lied in that the cells used were non-BMP7 gene-transfected stem cells induced by an active protein obtained in the step 4 (with a concentration of 1×10⁷ cells/ml).

The blank recombinant type III collagen 3D scaffold group: A blank recombinant type III collagen 3D scaffold containing no cells was prepared in a 12-well culture plate, the blank scaffold was transferred to a 6-well culture plate, a DMEM culture medium was added for culture under the same conditions, and morphological observation was performed on the 0th, 7th and 14th days of culture respectively. Additionally, the culture medium was collected, and the secretion amounts of type II collagen and glycosaminoglycans were determined by ELISA.

The gene of interest-transfected group: All operations for this group were basically the same as those of the "gene of interest-transfected + inducing factor group". The only difference lied in that the process of the step 4 (active protein-induced differentiation) was skipped. The BMP7 gene-transfected stem cells obtained in the step 3 were directly used for constructing and culturing the 3D scaffold in the step 5. All other operations, including scaffold construction, culture conditions, and detection time points, were the same as those of the "gene of interest-transfected + inducing factor group".

A method for preparing the recombinant type III collagen 3D scaffold containing 2×10⁶ stem cells included: 1 ml of a 200 mg/ml recombinant type III collagen solution (a solvent: physiological saline for injection) and 0.5 ml of a stem cell suspension with a concentration of 1×10⁷ cells/ml were added to a well of a 12-well plate; 0.8 ml of a 40 mg/ml fibrinogen solution was slowly added dropwise for mixing uniformly, and 0.2 ml of 1000 U/ml thrombin was added dropwise; a mixture was rapidly mixed uniformly on a shaker; the scaffold was placed in an incubator for 12 hours to initially solidify the scaffold; and the initially solidified scaffold was transferred to a 6-well culture plate and further cultured for 14 days. Finally, a recombinant type III collagen 3D culture scaffold containing approximately 2×10⁶ stem cells was obtained.

A method for preparing a blank recombinant type III collagen 3D culture scaffold was basically the same as the above method, and the only difference lied in that 0.5 ml of a 1×10⁷ cells/ml stem cell suspension was replaced with 0.5 ml of a PBS solution.

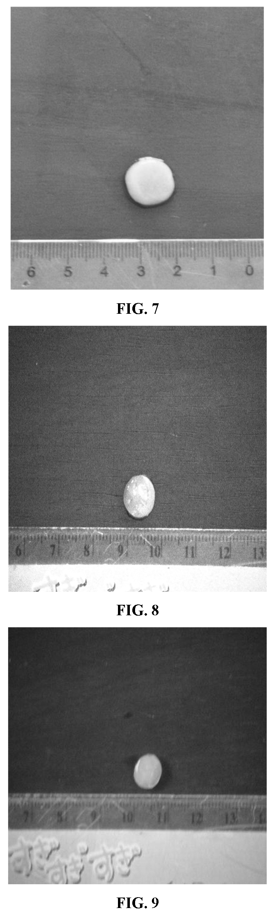

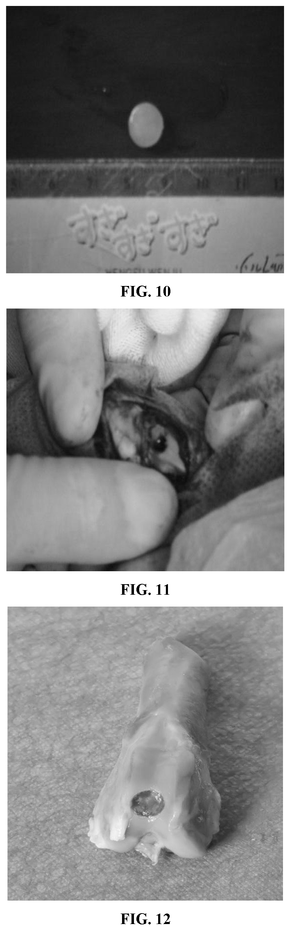

Experimental results: The blank recombinant type III collagen 3D scaffold group was dull white without cell filling (as shown in FIG. 7), and without secretion of type II collagen and glycosaminoglycan; cartilage from the BMP7 gene-transfected group was white and presented a cartilage-like tissue shape (as shown in FIG. 8), and the secretion amounts of type II collagen and glycosaminoglycans significantly increased; cartilage from the non-gene of interest-transfected + inducing factor group was white and presented a cartilage-like tissue shape (as shown in FIG. 9), and secretion levels of type II collagen and glycosaminoglycans were significantly higher than that of the BMP7 gene-transfected group; and the gene of interest-transfected + inducing factor group was white and presented a cartilage-like tissue shape and had a relatively dense structure (as shown in FIG. 10), and the secretion levels of type II collagen and glycosaminoglycans were significantly higher than those of the gene of interest-transfected group and the non-gene of interest-transfected + inducing factor group. The secretion levels of type II collagen and glycosaminoglycans were shown in Table 8 and Table 9. Therefore, adding inducing factors for the gene of interest-transfected group is an optimal method for constructing cartilage tissue.

**Table 8 Mean type II collagen secretion (ng/ml)**

| Group | 0 day | 7 days | 14 days |
|---|---|---|---|
| Blank recombinant type III collagen 3D scaffold group | 0 | 0 | 0 |
| Gene of interest-transfected group | 213.7 | 486.3 | 612.0 |
| Non-gene of interest-transfected + inducing factor group | 221.5 | 684.9 | 876.5 |
| Gene of interest-transfected + inducing factor group | 227.7 1 | 945.5 | 1028. 4 |

**Table 9 Mean glycosaminoglycan secretion of each group (µg/ml)**

| Group | 0 day | 7 days | 14 days |
|---|---|---|---|
| Blank recombinant type III collagen 3D scaffold group | 0 | 0 | 0 |
| Gene of interest-transfected group | 3.25 | 6.47 | 13.78 |
| Non-gene of interest-transfected + inducing factor group | 3.67 | 20.71 | 35.87 |
| Gene of interest-transfected group + inducing factor group | 3.89 | 28.40 | 50.16 |

### Example 2

In this example, an optimal technical route determined in Example 1 was used to construct a tissue-engineered cartilage graft. The route included: BMP7 gene-transfected stem cells were used, three active proteins (TGF-β 10.0 µg/L + PRP 1% + SOX9 2.5 µg/L) were combined for inducing, and a recombinant type III collagen 3D scaffold was constructed in vitro to finally form cartilage tissue (hereinafter referred to as a "BMP7 gene-transfected + mixed inducing factor group").

To evaluate effects, the following control groups were set up for this experiment, and an in vivo animal experiment was conducted:
A model group: Cartilage injury was induced without any treatment.

A blank recombinant type III collagen 3D scaffold group: Only a blank scaffold containing no cells was implanted.

A BMP7 gene-transfected group: Cartilage tissue constructed from BMP7 gene-transfected stem cells not induced by an active protein was implanted.

36 healthy male large-eared white rabbits with a body weight of 2.0-2.5 kg were randomly divided into 4 groups corresponding to the above four experimental groups respectively, where there were 9 rabbits in each group.

A method for creating a cartilage injury model included: A rabbit was administered intravenously (via an ear vein) with 1% sodium barbital at a dose of 30 mg/kg body weight, and after anesthesia, the rabbit was fixed on an operating table. An incision was made on a medial side of patella, the patella was dislocated laterally to expose articular cartilage on a patellar surface of a distal femur, an orthopedic hand drill was used to create a full-thickness cartilage defect (had a diameter of 5.0 mm, and reached a subchondral bone plate, with a small amount of blood exudation) on a surface of the articular cartilage, and tissue debris and blood clots were cleaned by rinsing with physiological saline to obtain a rabbit cartilage injury model (as shown in FIG. 11).

The model group: No treatment was performed on a cartilage injury model site of the rabbit;
the blank recombinant type III collagen 3D scaffold group: the cartilage injury model site was filled with a blank recombinant type III collagen 3D scaffold containing no cells; and
the BMP7 gene-transfected group: the cartilage injury model site was filled with cartilage tissue constructed from BMP7 gene-transfected stem cells and cultured in vitro for 14 days; a method for obtaining the constructed from BMP7 gene-transfected stem cells and cultured in vitro for 14 days was detailed as follows: a recombinant plasmid was constructed and the stem cells in the step 2 were transfected with reference to the step 1-3 of Example 1 to obtain BMP7 gene-transfected cells, and then the cells were directly constructed into a recombinant type III collagen 3D scaffold with reference to the method for the "gene of interest-transfected group" in the step 5 of Example 1, and cultured in vitro for 14 days to finally obtain required cartilage tissue (no active protein was added for induction in this process).

A BMP7 gene-transfected + mixed inducing factor group: The cartilage injury model site was filled with cartilage tissue constructed from BMP7 gene-transfected stem cells induced by an active protein and cultured in vitro for 14 days, and a specific method for preparing the cartilage tissue was the method for the "gene of interest-transfected + inducing factor group" described in the step 5 of Example 1.

After respective treatments for each experimental group, patella of each rabbit was reset, and the knee joint and skin of each rabbit were sutured. Postoperatively, the rabbits were returned to a cage for feeding, and hind limbs of the rabbits were not fixed, that is, the rabbits were allowed to move freely. Eight weeks later after the transplantation surgery, the rabbits were euthanized, and knee joints at transplantation sites were removed to observe and evaluate the repair of cartilage injury.

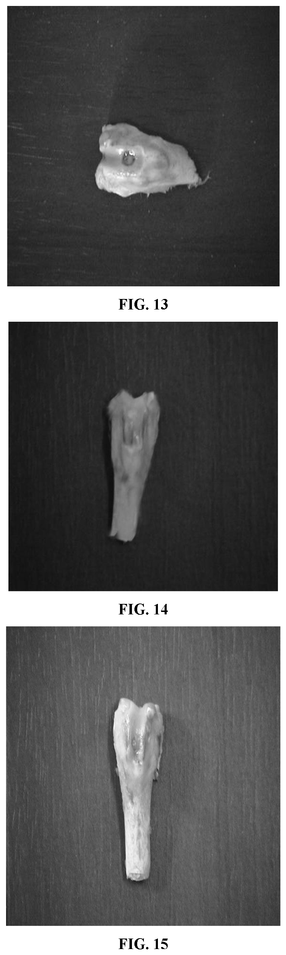

Results of repair at 8 weeks after the surgery were shown in FIGS. 12 to 15 (an effect diagram of only one rabbit in each experimental group was given, and the results of the same experimental group were roughly the same), indicating that: a bottom of cartilage defect of each rabbit in the model group was dark red, a wound surface thereof was concave and well-defined against normal tissue, cartilage injury of each of 9 animals was not repaired, and successful modeling was achieved (as shown in FIG. 12); in a blank 3D scaffold group, a filler was slightly lower than a normal joint surface, a graft site was centrally white, peripherally dark red, and well-defined against surrounding normal articular cartilage, and the cartilage injury of each of 9 animals was not repaired, indicating that the blank 3D scaffold had no repair effect on cartilage injury (as shown in FIG. 13); in the BMP7 gene-transfected group, a cartilage defect site of the rabbit was filled with a milky white graft that was color-lighter than the normal joint, a repaired tissue joint was level with a joint surface, without gaps or cracks, and integrated well with the surrounding tissue, and the cartilage injury of each of 9 animals was repaired, indicating that the BMP7 gene-transfected group had a significant repair effect on cartilage injury (as shown in FIG. 14); and in the BMP7 gene-transfected + mixed inducing factor group, a cartilage defect site of the rabbit was filled with a cartilage-like graft slightly different from the normal joint in color, a repaired tissue joint was level with a joint surface and well-defined against surrounding normal articular cartilage, without gaps or cracks, and was fully integrated with surrounding tissue, and the cartilage injury of each of 9 animals was repaired, indicating that the BMP7 gene-transfected + mixed inducing factor group had a significant repair effect on cartilage injury, and was superior to the BMP7 gene-transfected group (as shown in FIG. 15). The above results showed that cartilage defects of rabbits in the BMP7 gene-transfected + mixed inducing factor group were fully repaired, and this group was superior to other groups.

### Example 3

In this example, a method for constructing tissue-engineered cartilage using umbilical cord blood-derived mesenchymal stem cells is provided, and specifically includes:
step 1: stem cells were isolated and cultured; a specific process was the same as the step 2 in Example 1: stem cells were isolated and cultured; and
step 2: differentiation of the stem cells was induced using an active protein, with a specific process as follows:
   step 2-1: A 6-well culture plate was taken, and 2 ml of a cell suspension was added to each well. The cell suspension contained third-passage (P3) umbilical cord blood-derived mesenchymal stem cells with a concentration of 1×10⁵ cells/ml, the culture plate was placed in an incubator at 37°C with 5% CO₂ for culture, and after 24 hours of culture, the culture medium was discarded.
   Step 2-2: A DMEM culture medium containing active proteins (final concentration composition: TGF-β, 10 µg/L; PRP, 1%, v/v; and SOX9, 2.5 µg/L) was added to each well, and the cells were cultured at 37°C with 5% CO₂ for 3 passages (P4 to P6) for later use, and during the culture of each passage, replacement with a fresh DMEM culture medium of the same formula (containing the above active proteins) was performed once.
Step 3: The stem cells that had undergone induced differentiation culture (P3) in the step 2 were used to construct a recombinant type III collagen 3D culture scaffold in vitro, and a specific construction method was the same as that described in Example 1.
   Step 3-1: According to the "method for preparing the recombinant type III collagen 3D scaffold containing cells" described in the step 5 of Example 1, a 3D scaffold containing approximately 2×10⁶ cells per scaffold was constructed. This operation was performed in a 12-well plate.
   Step 3-2: The recombinant type III collagen 3D scaffold containing stem cells was transferred from a 12-well culture plate to a 6-well culture plate, 3 ml of a DMEM culture medium containing active proteins (final concentration composition: TGF-β1 (10.0 µg/L) + PRP (1%) + SOX9 (2.5 µg/L)) and 10% fetal bovine serum was added, the scaffold was cultured in an incubator at 37°C with 5% CO₂, replacement with a culture medium of the same formula was performed every 3 days, and after the above culture, tissue-engineered cartilage was finally obtained.

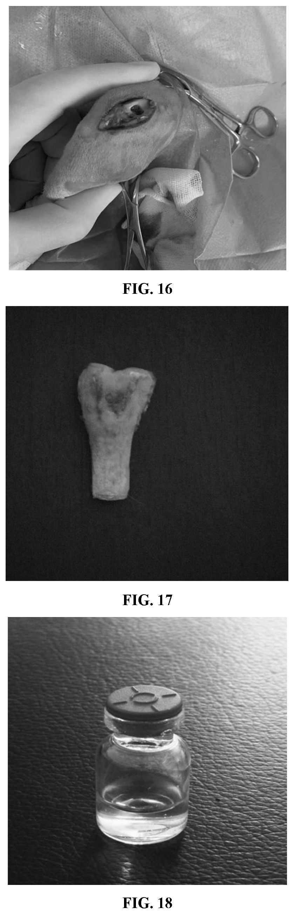

According to the method described in Example 2, a cartilage injury model was established for knee joints of each of 9 male rabbits (with a body weight of 2.0-2.5 kg), the model effect was shown in FIG. 16, and then the tissue-engineered cartilage was filled into a cartilage injury model site of each rabbit. Patella of each rabbit was reset, and the knee joint and skin of each rabbit were sutured. Postoperatively, the rabbits were returned to a cage for feeding, and hind limbs of the rabbits were not fixed, that is, the rabbits were allowed to move freely. Eight weeks later the transplantation, the rabbits were euthanized, and knee joints at transplantation sites were removed to evaluate the repair effect of cartilage injury.

FIG. 17 illustrates an effect of repair of tissue-engineered cartilage constructed in this example for 8 weeks, indicating that the tissue-engineered cartilage constructed using stem cells that were not genetically modified but were only induced by active proteins (TGF-β+ PRP +SOX9) still successfully repaired articular cartilage defects of rabbits, but the effect was slightly inferior to the repair effect of the tissue-engineered cartilage obtained by BMP7 gene transfection and active protein induction.

### Example 4

In this example, an optimal technical route determined in Example 1 was used, but a final product form was changed from "tissue-engineered cartilage" to "stem cell gel solution". The gel solution was prepared by mixing BMP7 gene-transfected stem cells induced by an active protein with an injectable gel carrier (hereinafter referred to as a "BMP7 gene-transfected + mixed inducing factor gel solution group").

A method for preparing the stem cell gel solution includes: The stem cells obtained in the step 4 (through BMP7 gene transfection + active protein induction) of Example 1 were added to physiological saline to prepare a cell suspension with a concentration of 5×10⁶ cells/ml, sodium hyaluronate was added to a 2.0% human albumin physiological saline solution to prepare a sodium hyaluronate gel solution, where a feeding ratio of sodium hyaluronate to 2% human albumin physiological saline was 1:30 (m/v), and the cell suspension was mixed with the sodium hyaluronate gel solution at a volume ratio of 1:1 to obtain the stem cell gel solution.

To evaluate effects, the following control groups were further set up for this experiment, and an in vivo animal experiment was conducted:
A model group: Cartilage injury was induced only without any treatment.

A blank gel solution group: An injury site was filled with a blank gel carrier containing no cells.

A BMP7 gene-transfected group: The injury site was filled with a gel solution prepared from BMP7 gene-transfected stem cells not induced by an active protein.

The BMP7 gene-transfected + mixed inducing factor gel solution group (experimental group): The injury site was filled with the gel solution prepared in this example.

36 healthy male large-eared white rabbits with a body weight of 2.0-2.5 kg were randomly divided into 4 groups corresponding to the above four experimental groups respectively, where there were 9 rabbits in each group.

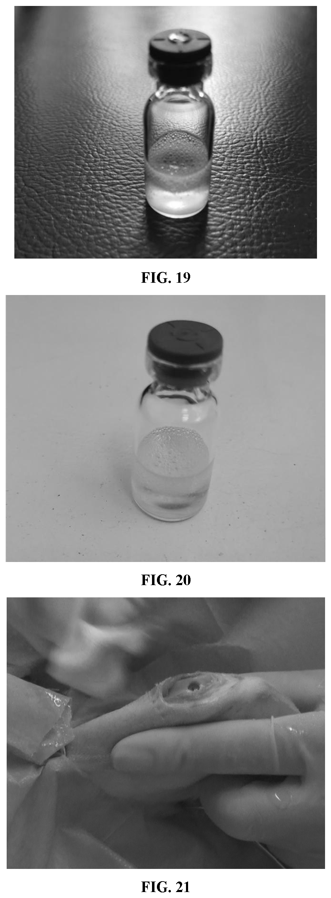

A method for creating a cartilage injury model included: The cartilage injury model was obtained according to the method in Example 2, with results shown in FIG. 21.

The model group: No treatment was performed on a cartilage injury model site of the rabbit; and
the blank gel solution group: the cartilage injury model site was filled with a blank gel solution containing no cells (as shown in FIG. 18), and the blank gel solution was prepared by mixing 2.0% human albumin-physiological saline and sodium hyaluronate with an equal amount of physiological saline.

The BMP7 gene-transfected group: The cartilage injury model site was filled with a stem cell gel solution prepared from BMP7 gene-transfected stem cells that were not induced by an active protein but cultured in vitro for 14 days; and a method for obtaining the stem cell gel solution was detailed as follows: a recombinant plasmid was constructed and stem cells were transfected with reference to the step 1-3 of Example 1 to obtain BMP7 gene-transfected cells, after the stem cells were cultured in vitro for 14 days (no active protein was added for induction in this process), the stem cells were collected and suspended in physiological saline, and then, the cell suspension was mixed with the 2.0% human albumin-physiological saline-containing sodium hyaluronate solution in equal amounts to obtain the stem cell gel solution (as shown in FIG. 19).

A BMP7 gene-transfected + mixed inducing factor group: The cartilage injury model site was filled with a gel solution prepared from BMP7-gene transfected stem cells that were induced by an active protein and cultured in vitro for 14 days; and a method for obtaining the stem cell gel solution was detailed as follows: with reference to the method for the "gene of interest-transfected + inducing factor group" in Example 1, the BMP7 gene-transfected stem cells were induced by an active protein and cultured in vitro for 14 days, then the cells were collected and suspended in physiological saline, and the cell suspension was mixed with the 2.0% human albumin-physiological saline-containing sodium hyaluronate solution in equal amounts to obtain the stem cell gel solution (as shown in FIG. 20).

After respective treatments for each experimental group, patella of each rabbit was reset, and the knee joint and skin of each rabbit were sutured. Postoperatively, the rabbits were returned to a cage for feeding, and hind limbs of the rabbits were not fixed, that is, the rabbits were allowed to move freely. Eight weeks later the transplantation, the rabbits were euthanized, and joints at transplantation sites were removed to evaluate the repair effect of cartilage injury.

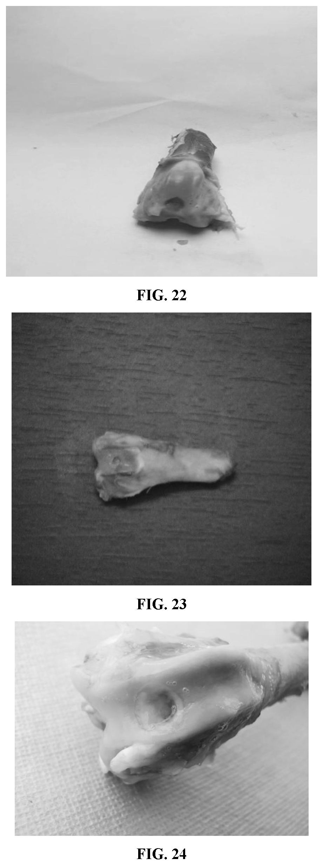

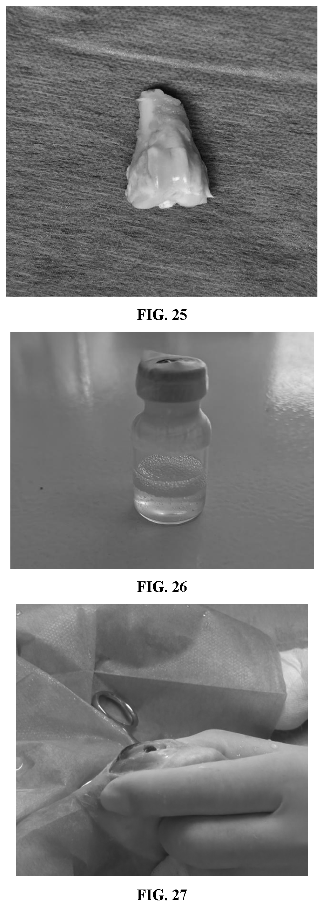

Results of repair at 8 weeks after the surgery were shown in FIGS. 22 to 25 (an effect diagram of only one rabbit in each experimental group was given, and the results of the same experimental group were not significantly different), indicating that: a bottom of cartilage defect of each rabbit in the model group was dark red, a wound surface thereof was concave without tissue filling, cartilage injury of each of 9 animals was not repaired (as shown in FIG. 22); in the blank gel solution group, a filler was lower than a normal joint surface, a graft site was centrally white, peripherally dark red, and well-defined against surrounding normal articular cartilage, and the cartilage injury of each of 9 animals was not repaired, indicating that the blank gel solution had no repair effect on cartilage injury (as shown in FIG. 23); in the BMP7 gene-transfected group, a cartilage defect site of the rabbit was filled with a milky white graft that was color-lighter than the normal joint, a repaired tissue joint was level with a joint surface, without gaps or cracks, and integrated well with the surrounding tissue, and the cartilage injury of each of 9 animals was repaired, indicating that the BMP7 gene-transfected group had a significant repair effect on cartilage injury (as shown in FIG. 24); and in the BMP7 gene-transfected + mixed inducing factor group, a cartilage defect site of the rabbit was filled with a cartilage-like graft slightly different from the normal joint in color, a repaired tissue joint was level with a joint surface and well-defined against surrounding normal articular cartilage, without gaps or cracks, and was fully integrated with surrounding tissue, and the cartilage injury of each of 9 animals was repaired, indicating that the BMP7 gene-transfected + mixed inducing factor group had a significant repair effect on cartilage injury, and was superior to the BMP7 gene-transfected group (as shown in FIG. 25). The above results showed that cartilage defects of rabbits in the BMP7 gene-transfected + mixed inducing factor group were fully repaired, and this group was superior to other groups in the repair effect.

### Example 5

In this example, a method for preparing a stem cell gel solution using umbilical cord blood-derived mesenchymal stem cells is provided, and specifically includes:
step 1: isolation, primary culture, and subculture of umbilical cord blood-derived mesenchymal stem cells were performed according to the method described in the step 1 of Example 3;
step 2: differentiation culture of obtained P3 stem cells were induced with mixed active proteins of optimal concentrations (TGF-β 10 µg/L + PRP 1% + SOX9 2.5 µg/L), and the cells were cultured for 3 passages consecutively according to the method described in the step 2 of Example 3; and
step 3: a method for preparing a stem cell gel solution in vitro using the stem cells cultured for 3 passages in the step 2 includes: the cells were resuspended in physiological saline to prepare a cell suspension with a concentration of 5×10⁶ cells/ml, a sodium hyaluronate gel solution was prepared using a 2.0% human albumin physiological saline solution, where a feeding ratio of sodium hyaluronate to 2% human albumin physiological saline was 1:30 (mass/volume), the sodium hyaluronate gel solution was mixed with the cell suspension at a volume ratio of 1:1 (v/v) to prepare a stem cell gel solution, and finally, the stem cell gel solution with a cell count of approximately 2.5×10⁶ cells/mL was obtained (as shown in FIG. 26).

According to the method described in Example 2, a cartilage injury model was established for knee joints of each of 9 male rabbits (with a body weight of 2.0-2.5 kg), the effect was shown in FIG. 27, a cartilage injury model site of each rabbit was filled with the stem cell gel solution, and then patella of each rabbit was reset, and the knee joint and skin of each rabbit were sutured. Postoperatively, the rabbits were returned to a cage for feeding, and hind limbs of the rabbits were not fixed, that is, the rabbits were allowed to move freely. Eight weeks later the transplantation, the rabbits were euthanized, and knee joints at transplantation sites were removed to evaluate the repair effect of cartilage injury.

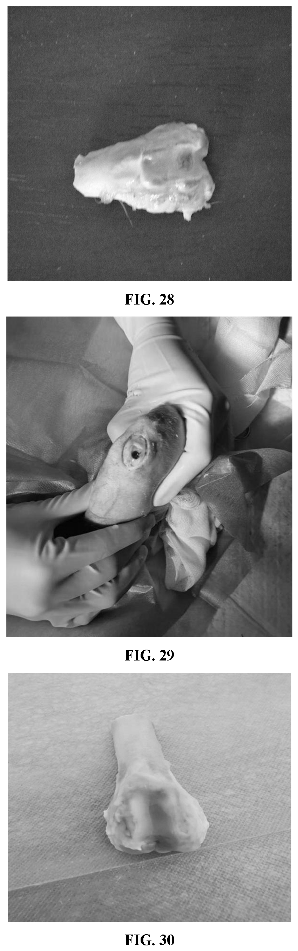

FIG. 28 illustrates a repair effect of the stem cell gel solution prepared in this example after 8 weeks of transplantation, indicating that the stem cell gel solution prepared from stem cells that were not genetically modified but were only induced by active proteins still successfully repaired articular cartilage defects of rabbits, but the effect was slightly inferior to the repair effect of the stem cell gel solution obtained by BMP7 gene transfection and active protein induction.

### Example 6

In this example, a method for preparing a stem cell gel solution or constructing tissue-engineered cartilage using bone marrow-derived mesenchymal stem cells transfected with a gene of interest BMP7 is provided, and specifically includes:
step 1: a recombinant plasmid containing the gene of interest BMP7 (pcDNA3.1-BMP7) was constructed according to the method described in the step 1 of Example 1.
step 2: stem cells were isolated and cultured, specifically including: 2-3 ml of bone marrow was collected by puncturing a proximal femur of rabbit with a bone marrow puncture needle. The collected bone marrow served as a starting material, and isolation, primary culture, and subculture of bone marrow-derived mesenchymal stem cells were performed fully according to the method in the step 2 (isolation and culture of umbilical cord blood-derived mesenchymal stem cells) in Example 1 to obtain a sufficient number of P3 cells.
Step 3: According to the method in the step 3 of Example 1, the pcDNA3.1-BMP7 plasmid constructed in the step 1 was used to transfect P3 bone marrow-derived mesenchymal stem cells cultured in the step 2 and obtain the BMP7 gene-transfected bone marrow-derived mesenchymal stem cells.
step 4: differentiation culture of stem cells transfected with the gene of interest was induced with an active protein; operation was performed according to the step 4 in Example 1, specifically including: a 96-well culture plate was taken, 100 µl of stem cells transfected with the pcDNA3.1-BMP7 plasmid was added to each well at 1×10⁵ cells/ml, the culture plate was placed in an incubator at 37°C with 5% CO₂ for culture, and after 24 hours of culture, a culture medium was discarded, and a DMEM culture medium containing an active protein was added. The DMEM culture medium containing an active protein included the following components of final concentrations: TGF-β, 10 µg/L; PRP, 1%, v/v; SOX9, 2.5 µg/L; the DMEM served as a basic medium.
step 5: BMP7 gene-transfected bone marrow-derived mesenchymal stem cells that had undergone induced differentiation in the step 4 were collected, and these cells were prepared into a stem cell gel solution according to the method in the step 3 of Example 5; and
step 6: BMP7 gene-transfected bone marrow-derived mesenchymal stem cells that had undergone induced differentiation in the step 4 were collected, a recombinant type III collagen 3D culture scaffold was constructed in vitro using the stem cells according to the method in the step 5 of Example 1, and long-term culture was performed to finally form tissue-engineered cartilage.

According to the method in Example 2, a cartilage injury model was established for knee joints of each of 18 male rabbits (with a body weight of 2.0-2.5 kg), and the effect was shown in FIG. 29; and the 18 rabbits were divided into two groups: a gel solution group (9 rabbits): an injury site was filled with the stem cell gel solution prepared in the step 5 of this example (referring to Example 4 for details about a transplantation method); and a tissue-engineered cartilage group (9 rabbits): an injury site was filled with the tissue-engineered cartilage constructed in the step 6 of this example (referring to Example 2 for details about a transplantation method).

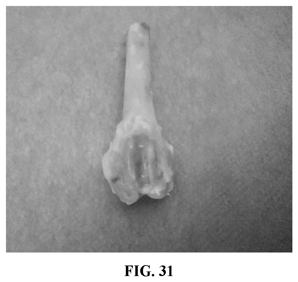

FIG. 30 illustrates an effect of repair for 8 weeks with the stem cell gel solution obtained in this example, and FIG. 31 illustrates an effect of repair for 8 weeks with the tissue-engineered cartilage obtained in this example, indicating that articular cartilage defects of rabbits were successfully repaired by both the stem cell gel solution and the constructed tissue-engineered cartilage prepared in this example; and therefore, both bone marrow-derived and umbilical cord blood-derived stem cells may be used for preparing the stem cell gel solution and constructing tissue-engineered cartilage in the present invention.

The examples mentioned above are merely several embodiments of the present invention, and are specifically described in details, but may not be interpreted as limiting the scope of the patent for the present invention as a result. It should be noted that for those of ordinary skill in the art, they may also make several transformations and improvements on the premise of not deviating from the conception of the present invention, and these transformations and improvements shall fall within the scope of protection of the present invention. The scope of protection of the present patent for disclosure shall be governed by the appended claims.

## Claims

1. An application of stem cells or transgenic stem cells in preparing a stem cell gel solution or tissue-engineered cartilage.

2. The application according to claim 1, **characterized in that** the stem cells are derived from umbilical cord blood, umbilical cords, placentas, bone marrow, or fat; and/or, a gene in transgenic stem cells is selected from at least one of a bone morphogenetic protein (BMP) gene, a transforming growth factor-β (TGF-β) gene, and a SOX9 gene.

3. The application according to claim 1 or 2, **characterized in that** the method for preparing a stem cell gel solution or constructing tissue-engineered cartilage using stem cells comprises:
step 1: isolating and culturing stem cells;
step 2: inducing differentiation of the stem cells using an active protein; and
step 3: preparing a stem cell gel solution using the differentiated stem cells, or constructing a type III collagen 3D scaffold in vitro using the stem cells obtained in the step 2 to finally form tissue-engineered cartilage.

4. The application according to claim 1 or 2, **characterized in that** a method for preparing a stem cell gel solution or constructing tissue-engineered cartilage using transgenic stem cells comprises:
step 1: constructing a recombinant plasmid containing a gene of interest;
step 2: isolating and culturing stem cells;
step 3: transfecting the stem cells cultured in the step 2 with the recombinant plasmid constructed in the step 1 to obtain stem cells transfected with the gene of interest;
step 4: inducing differentiation of the stem cells transfected with the gene of interest using an active protein; and
step 5: preparing a stem cell gel solution using the differentiated stem cells, or constructing a type III collagen 3D scaffold in vitro using the stem cells obtained in the step 4 to finally form tissue-engineered cartilage.

5. The application according to claim 4, **characterized in that** the constructing a recombinant plasmid containing a gene of interest in the step 1 comprises:
step 1-1: obtaining a pBlue-gene of interest plasmid;
step 1-2: treating the plasmid with double digestion, then extracting and purifying a gene fragment;
step 1-3: similarly treating a pcDNA3.1 vector with double digestion, and then extracting and purifying the vector fragment;
step 1-4: ligating the purified gene fragment and the vector fragment to form a pcDNA3.1-gene of interest vector plasmid; and
step 1-5: introducing the plasmid into DH5α competent cells for amplification, and finally extracting and purifying an amplified product to obtain the required plasmid.

6. The application according to claim 3, **characterized in that** the inducing differentiation culture of the stem cells using an active protein in the step 2 comprises:
adding an active protein into a differentiation culture medium containing stem cells or stem cells transfected with the gene of interest, culturing at 37°C with 5% CO₂, culturing for 3-5 passages for later use, and replacing with the same differentiation culture medium per passage of culture.

7. The application according to claim 4, **characterized in that** the inducing differentiation culture of the stem cells transfected with the gene of interest using an active protein in the step 4 comprises:
adding an active protein into a differentiation culture medium containing stem cells or stem cells transfected with the gene of interest, culturing at 37°C with 5% CO₂, culturing for 3-5 passages for later use, and replacing with the same differentiation culture medium per passage of culture.

8. The application according to claim 6 or 7, **characterized in that** the active protein comprises at least one of the following components: TGF-β, PRP, and SOX9.

9. The application according to claim 8, **characterized in that** the active protein is composed of TGF-β, PRP, and SOX9, and final concentrations of each component in a differentiation culture system are as follows: TGF-β, 4.0-16.0 µg/L; PRP, 0.5-2.0%, v/v; and SOX9, 2.5-10 µg/L.

10. The application according to claim 3, **characterized in that** the preparing a stem cell gel solution using the stem cells in the step 3 comprises:
mixing stem cells with physiological saline to prepare a cell suspension with a concentration of 0.5×10⁷-1×10⁷ cells/ml, adding sodium hyaluronate to a 1.5%-2.5% (mass concentration) albumin physiological saline solution to prepare a sodium hyaluronate gel solution, and mixing the sodium hyaluronate gel solution with the cell suspension at a volume ratio of 1:0.5-2 to obtain a stem cell gel solution.

11. The application according to claim 4, **characterized in that** the preparing a stem cell gel solution using the stem cells in the step 5 comprises:
mixing stem cells with physiological saline to prepare a cell suspension with a concentration of 0.5×10⁷-1×10⁷ cells/ml, adding sodium hyaluronate to a 1.5%-2.5% (mass concentration) albumin physiological saline solution to prepare a sodium hyaluronate gel solution, and mixing the sodium hyaluronate gel solution with the cell suspension at a volume ratio of 1:0.5-2 to obtain a stem cell gel solution.

12. The application according to claim 3, **characterized in that** the constructing a type III collagen 3D scaffold in vitro using the stem cells to finally form tissue-engineered cartilage in the step 3 comprises:
preparing a type III collagen 3D scaffold using the differentiated stem cells (2.5×10⁶-5×10⁶ stem cells per scaffold), adding a DMEM cell culture medium containing the active protein to the scaffold, culturing at 37°C with 5% CO₂, replacing the DMEM cell culture medium containing the active protein every 7 days, and continuously culturing for 20-24 days to obtain tissue-engineered cartilage.

13. The application according to claim 4, **characterized in that** the constructing a type III collagen 3D scaffold in vitro using the stem cells to finally form tissue-engineered cartilage in the step 5 comprises:
preparing a type III collagen 3D scaffold using the differentiated stem cells (2.5×10⁶-5×10⁶ stem cells per scaffold), adding a DMEM cell culture medium containing the active protein to the scaffold, culturing at 37°C with 5% CO₂, replacing the DMEM cell culture medium containing the active protein every 7 days, and continuously culturing for 20-24 days to obtain tissue-engineered cartilage.

14. The application according to claim 12 or 13, **characterized in that** a method for preparing the type III collagen 3D scaffold comprises: mixing a type III collagen solution with a stem cell suspension, adding a fibrinogen solution and thrombin sequentially, mixing uniformly, allowing to stand for 8-12 h, and then culturing at 37°C with 5% CO₂ for 14-21 days to obtain a cell-containing 3D scaffold; and/or
final concentrations of components of the active protein in the DMEM cell culture medium are as follows: TGF-β, 4.0-16.0 µg/L; PRP, 0.5-2.0%, v/v; and SOX9, 2.5-10 µg/L; and preferably, final concentrations of components of the active protein in the DMEM cell culture medium are as follows: TGF-β, 10 µg/L; PRP, 1%, v/v; and SOX9, 2.5 µg/L.

15. A stem cell gel solution or tissue-engineered cartilage obtained by the application according to any one of claims 1 to 14.

16. An application of the stem cell gel solution or tissue-engineered cartilage according to claim 15 in preparing a bone graft.
